(19) Europäisches Patentamt
European Patent Office
Office européen des brevets

(11) Veröffentlichungsnummer: **0 161 604**
**A2**

(12) # EUROPÄISCHE PATENTANMELDUNG

(21) Anmeldenummer: **85105485.8**

(22) Anmeldetag: **06.05.85**

(51) Int. Cl.⁴: **C 07 D 223/16**, C 07 D 243/04, A 61 K 31/55 // C07C59/72, C07C57/58, C07C79/38, C07C103/30, C07C49/755, C07C93/14, C07C121/43

(30) Priorität: **17.05.84 DE 3418270**

(43) Veröffentlichungstag der Anmeldung: **21.11.85 Patentblatt 85/47**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Reiffen, Manfred, Dr. Dipl.-Chem., Matthias-Erzberger-Strasse 40, D-7950 Biberach 1 (DE)**
Erfinder: **Heider, Joachim, Dr. Dipl.-Chem., Am Hang 3, D-7951 Warthausen 1 (DE)**
Erfinder: **Austel, Volkhard, Dr. Dipl.-Chem., Kapellenweg 7, D-7950 Biberach 1 (DE)**
Erfinder: **Hauel, Norbert, Dr. Dipl.-Chem., Händelstrasse 12, D-7950 Biberach 1 (DE)**
Erfinder: **Kobinger, Walter, Prof. Dr., Belghofergasse 27, A-1121 Wien (AT)**
Erfinder: **Lillie, Christian, Dr., Hansl-Niese-Weg 12, A-1130 Wien (AT)**

(54) **Neue Aminotetralinderivate, diese Verbindungen enthaltende Arzneimittel und Verfahren zu ihrer Herstellung.**

(57) Die vorliegende Erfindung betrifft neue Aminotetralinderivate der allgemeinen Formel

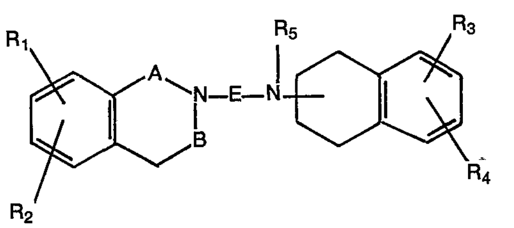

,(I)

in der
A eine $-CH_2-CH_2-$, $-CH=CH-$, $-NH-CO-$ oder $-CH_2-CO-$Gruppe
und
B eine Methylen-, Carbonyl- oder Thiocarbonylgruppe oder
A eine $-CO-CO-$ oder $-CH(OH)-CO-$Gruppe
und B eine Methylengruppe,
E eine gegebenenfalls durch eine Alkylgruppe substituierte geradkettige Alkylengruppe, eine 2-Hydroxy-n-propylen-, 2-Hydroxy-n-butylen- oder 3-Hydroxy-n-butylengruppe,
$R_1$ ein Wasserstoff-, Fluor-, Chlor- oder Bromatom, eine Trifluormethyl-, Nitro-, Amino-, Alkylamino-, Dialkylamino-, Alkyl-, Alkylthio-, Hydroxy-, Alkoxy- oder Phenylalkoxygruppe,
$R_2$ ein Wasserstoff-, Chlor- oder Bromatom, eine Hydroxy-, Alkoxy-, Phenylalkoxy- oder Alkylgruppe, oder
$R_1$ und $R_2$ zusammen eine Alkylendioxygruppe,
$R_3$ und $R_4$, die gleich oder verschieden sein können, Wasserstoff-, Fluor-, Chlor- oder Bromatome, Alkyl-, Hydroxy-, Alkoxy-, Nitro-, Amino-, Alkylamino- oder Dialkylaminogruppen oder zusammen eine Methylendioxygruppe, und
$R_5$ ein Wasserstoffatom, eine Alkenylgruppe, eine Alkyl- oder Phenylalkylgruppe, bedeuten, und deren Säureadditionssalze mit anorganischen oder organischen Säuren, welche wertvolle pharmakologische Eigenschaften aufweisen, insbesondere eine herzfrequenzsenkende Wirkung.

Die neuen Verbindungen können nach an sich bekannten Verfahren hergestellt werden.

0161604

DR. KARL THOMAE GMBH
D-7950 Biberach 1

Case 5/906
Dr. Fl/Kp

# Neue Aminotetralinderivate, diese Verbindungen enthaltende Arzneimittel und Verfahren zu ihrer Herstellung

Gegenstand der vorliegenden Erfindung sind neue Aminotetralinderivate der allgemeinen Formel

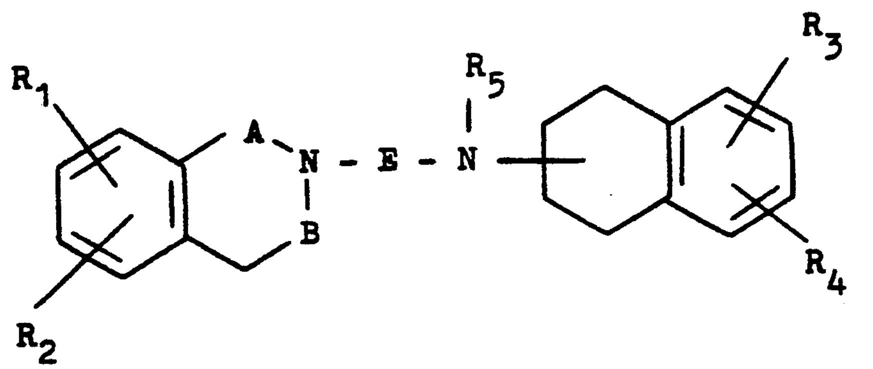

,(I)

deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

Die neuen Verbindungen weisen wertvolle pharmakologische Eigenschaften auf, insbesondere eine langanhaltende herzfrequenzsenkende Wirkung und eine herabsetzende Wirkung auf den $O_2$-Bedarf des Herzens.

In der obigen allgemeinen Formel I bedeutet

A eine $-CH_2-CH_2-$, $-CH=CH-$, $-\underset{5}{NH}-CO-$ oder $-\underset{5}{CH_2}-CO-$Gruppe

und

B eine Methylen-, Carbonyl- oder Thiocarbonylgruppe oder

A eine -CO-CO- oder $-\underset{5}{\overset{OH}{CH}}-CO-$Gruppe und B eine Methylengruppe,

E eine gegebenenfalls durch eine Alkylgruppe mit 1 bis 3 Kohlenstoffatomen substituierte geradkettige Alkylengruppe mit 2 bis 4 Kohlenstoffatomen, eine 2-Hydroxy-n-propylen-, 2-Hydroxy-n-butylen- oder 3-Hydroxy-n-butylengruppe,

$R_1$ ein Wasserstoff-, Fluor-, Chlor- oder Bromatom, eine Trifluormethyl-, Nitro-, Amino-, Alkylamino-, Dialkylamino-, Alkyl-, Alkylthio-, Hydroxy-, Alkoxy- oder Phenylalkoxygruppe, wobei jeder Alkylteil jeweils 1 bis 3 Kohlenstoffatome enthalten kann,

$R_2$ ein Wasserstoff-, Chlor- oder Bromatom, eine Hydroxy-, Alkoxy-, Phenylalkoxy- oder Alkylgruppe, wobei jeder Alkylteil jeweils 1 bis 3 Kohlenstoffatome enthalten kann, oder

$R_1$ und $R_2$ zusammen eine Alkylendioxygruppe mit 1 oder 2 Kohlenstoffatomen,

$R_3$ und $R_4$, die gleich oder verschieden sein können, Wasserstoff-, Fluor-, Chlor- oder Bromatome, Alkyl-, Hydroxy-, Alkoxy-, Nitro-, Amino-, Alkylamino- oder Dialkylaminogruppen oder zusammen eine Methylendioxygruppe, wobei jeder Alkylteil 1 bis 3 Kohlenstoffatome enthalten kann, und

$R_5$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatomen enthalten kann.

Für die bei der Definition der Reste eingangs erwähnten Bedeutungen kommt beispielsweise

für $R_1$ die des Wasserstoff-, Fluor-, Chlor- oder Bromatoms, der Methyl-, Ethyl-, n-Propyl-, Isopropyl-, Trifluormethyl-, Hydroxy-, Methoxy-, Ethoxy-, n-Propoxy-, Isopropoxy-, Methylthio-, Ethylthio-, Isopropylthio-, Nitro-, Amino-, Methylamino-, Ethylamino-, n-Propylamino-, Isopropylamino-, Dimethylamino-, Diethylamino-, Di-n-propylamino-, Diisopropylamino-, Methyl-ethylamino-, Methyl-n-propylamino-, Methyl-isopropylamino-, Ethyl-n-propylamino-, Benzyloxy-, 1-Phenylethoxy-, 1-Phenylpropoxy-, 2-Phenylethoxy- oder 3-Phenylpropoxygruppe,

für $R_2$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, 1-Phenylethoxy-, 2-Phenylethoxy-, 2-Phenylpropoxy- oder 3-Phenylpropoxygruppe oder zusammen mit $R_1$ die der Methylendioxy- oder Ethylendioxygruppe,

für $R_3$ und $R_4$, die gleich oder verschieden sein können, jeweils die des Wasserstoff-, Fluor-, Chlor- oder Bromatoms, der Methyl-, Ethyl-, n-Propyl-, Isopropyl-, Hydroxy-, Methoxy-, Ethoxy-, n-Propoxy-, Isopropxy-, Amino-, Methylamino-, Ethylamino-, n-Propylamino-, Isopropylamino-, Dimethylamino-, Diethylamino-, Di-n-propylamino-, Diisopropylamino- oder N-Ethyl-methylaminogruppe,

für $R_5$ die des Wasserstoffatoms, der Methyl-, Ethyl-, n-Propyl-, Isopropyl-, Benzyl-, 1-Phenylethyl-, 2-Phenylethyl-, 1-Phenylpropyl-, 1-Methyl-1-phenylethyl-, 3-Phenyl-

propyl-, Allyl-, n-Buten-(2)-yl- oder n-Penten-(2)-ylgruppe und

für E die der Ethylen-, n-Propylen-, n-Butylen-, 1-Methylethylen-, 2-Ethyl-ethylen-, 1-Propyl-ethylen-, 1-Methyl-n-propylen-, 2-Methyl-n-propylen-, 1-Ethyl-n-propylen-, 3-Ethyl-n-propylen-, 2-Propyl-n-propylen-, 2-Methyl-n-butylen-, 2-Hydroxy-n-propylen-, 2-Hydroxy-n-butylen- oder 3-Hydroxy-n-butylengruppe.

Bevorzugte Verbindungen sind jedoch die Verbindungen der allgemeinen Formel

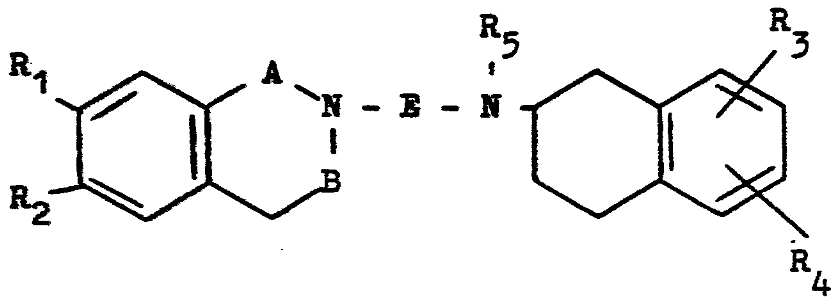

,(Ia)

in der

A eine $-CH_2-CH_2-$, $-CH=CH-$, $-NH-CO-$, $-CH_2-CO-$, $-CO-CO-$

oder $-\underset{5}{\overset{OH}{CH}}-CO$-Gruppe und B eine Methylengruppe oder

A eine $-CH_2-CH_2-$ oder $-CH=CH$-Gruppe und B eine Carbonyl- oder Thiocarbonylgruppe sind,

E eine n-Propylengruppe,

$R_1$ ein Chloratom- oder Bromatom, eine Methyl-, Methoxy-, Nitro-, Amino-, Methylamino- oder Dimethylaminogruppe,

$R_2$ ein Chlor- oder Bromatom, eine Methyl- oder Methoxygruppe oder $R_1$ und $R_2$ zusammen eine Methylendioxy- oder Ethylendioxygruppe,

$R_3$ ein Wasserstoff-, Chlor- oder Bromatom, eine Hydroxy-, Methoxy-, Amino-, Methylamino- oder Dimethylaminogruppe,

$R_4$ ein Wasserstoff- oder Chloratom oder eine Methoxygruppe zusammen mit $R_3$ eine Methylendioxygruppe und und

$R_5$ ein Wasserstoffatom, eine Methyl- oder Allylgruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel Ia sind jedoch diejenigen, in denen

A eine $-CH_2-CH_2-$ und B eine Carbonyl- oder Thiocarbonylgruppe oder

A eine -NH-CO-Gruppe und

B eine Methylengruppe,

E eine n-Propylengruppe,

$R_1$ und $R_2$ jeweils eine Methoxygruppe oder $R_1$ und $R_2$ zusammen eine Methylendioxygruppe,

$R_3$ und $R_4$ jeweils eine Methoxygruppe oder $R_3$ und $R_4$ zusammen eine Methylendioxygruppe und

$R_5$ ein Wasserstoffatom oder eine Methylgruppe bedeuten, und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Umsetzung einer Verbindung der allgemeinen Formel

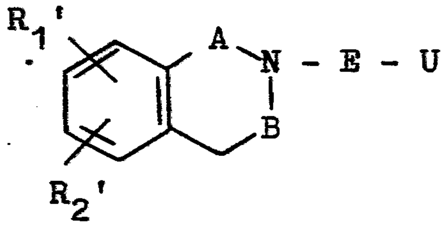

,(II)

mit einer Verbindung der allgmeinen Formel

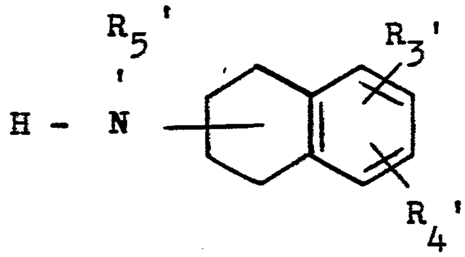

,(III)

in der

A, B und E wie eingangs definiert sind,

$R_1$' eine durch einen Schutzrest geschützte Hydroxy-, Amino- oder Alkylaminogruppe darstellt oder die für $R_1$ eingangs erwähnten Bedeutungen besitzt,

$R_2$' eine durch einen Schutzrest geschützte Hydroxygruppe darstellt oder die für $R_2$ eingangs erwähnten Bedeutungen besitzt,

$R_3$' eine durch einen Schutzrest geschützte Hydroxy-, Amino- oder Alkylaminogruppe darstellt oder die für $R_3$ eingangs erwähnten Bedeutungen besitzt,

$R_4$' eine durch einen Schutzrest geschützte Hydroxy-, Amino- oder Alkylaminogruppe darstellt oder die für $R_4$ eingangs erwähnten Bedeutungen besitzt,

$R_5$' eine Schutzgruppe für eine Aminogruppe darstellt oder die für $R_5$ eingangs erwähnten Bedeutungen besitzt, und

U eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und

als Schutzrest für eine Amino- oder Alkylaminogruppe die Acetyl-, Benzoyl-, Ethoxycaronyl- oder Benzylgruppe in Betracht.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydris wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel III durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temeraturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b) Zur Herstellung von Verbindungen der allgemeinen Formel I, in der A die $-CH_2-CH_2-$Gruppe, B die Methylen- oder Carbonylgruppe und $R_5$ keine Alkenylgruppe mit 3 bis 5 Kohlenstoffatomen darstellen:

Hydrierung einer Verbindung der allgemeinen Formel

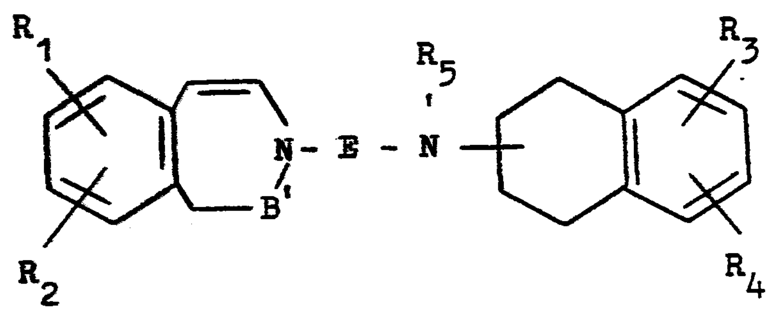

,(IV)

in der
$R_1$ bis $R_5$ und E wie eingangs definiert sind und
B' die Methylen- oder Carbonylgruppe darstellt.

Die Hydrierung wird in einem Lösungsmittel oder Lösungsmittelgemisch wie Methanol, Ethanol, Essigsäureethylester oder Eisessig mit katalytisch angeregtem Wasserstoff, z.B. mit Wasserstoff in Gegenwart von Platin oder Palladium/Kohle, bei einem Wasserstoffdruck von 1 bis 7 bar, vorzugsweise jedoch von 3 bis 5 bar, und bei Temperaturen zwischen 0 und 75°C, vorzugsweise jedoch bei Temeraturen zwischen 20 und 50°C, durchgeführt.

Bedeutet in einer Verbindung der allgemeinen Formel I $R_5$ eine Alkenylgruppe, so wird diese bei der Reduktion gleichzeitig in die entsprechende Alkylgruppe bzw. $R_1$ und/oder $R_2$ eine Benzyloxygruppe, so wird diese bei der Reduktion in die entsprechende Hydroxygruppe überführt.

c) Zur Herstellung von Verbindungen der allgemeinen Formel I, in der B eine Thiocarbonylgruppe darstellt:

Umsetzung einer Verbindung der allgemeinen Formel

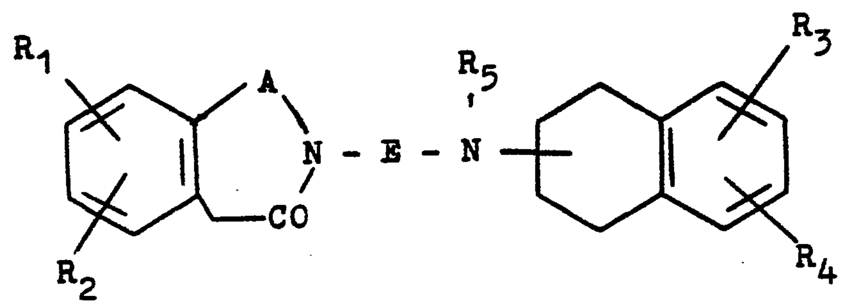

,(V)

in der
$R_1$ bis $R_5$, A und E wie eingangs definiert sind, mit einem schwefeleinführenden Mittel.

Die Umsetzung wird in einem schwefeleinführenden Mittel wie Phosphorpentasulfid oder 2,4-Bis(4-methoxyphenyl)-1,3-dithia-2,4-diphosphetan-2,4-disulfid zweckmäßigerweise in einem Lösungsmittel wie Toluol oder Xylol bei Temperaturen zwischen 50 und 150°C, z.B. bei der Siedetemperatur des Reaktionsgemisches, durchgeführt.

d) Zur Herstellung von Verbindungen der allgemeinen Formel I, in der A eine $-\overset{OH}{\overset{|}{C}}H-CO-$Gruppe darstellt:

Reduktion einer Verbindung der allgemeinen Formel

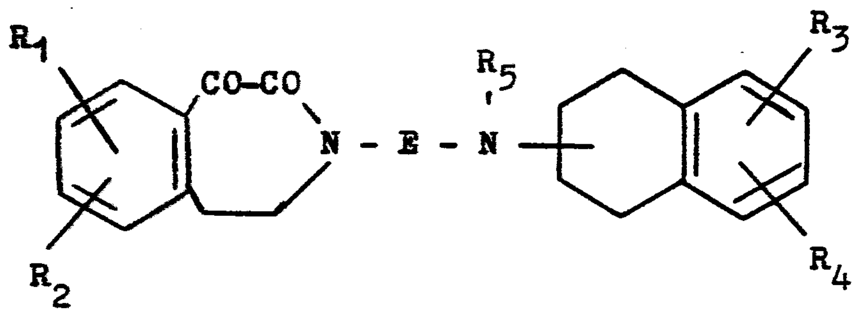

,(VI)

in der
$R_1$ bis $R_5$ und E wie eingangs definiert sind.

Die Umsetzung wird in Gegenwart eines geeigneten Reduktionsmittels wie einem Metallhydrid, z.B. Natriumborhydrid, in einem geeigneten Lösungsmittel wie Wasser/Methanol oder Methanol/Ether, bei Temperaturen zwischen 0 und 80°C, vorzugsweise jedoch bei Temperaturen zwischen 15 und 40°C, durchgeführt.

e) Zur Herstellung von Verbindungen der allgemeinen Formel I, in der A eine $-CH_2-CH_2-$ oder $-CH=CH-$ und B eine Methylengruppe darstellen:

Reduktion einer Verbindung der allgemeinen Formel

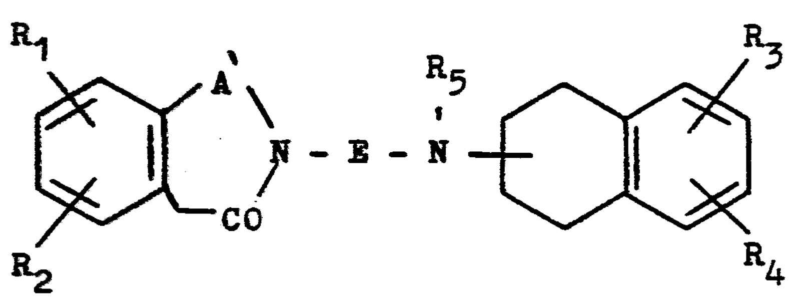

,(VII)

in der
$R_1$ bis $R_5$ und E wie eingangs definiert sind und
A' eine $-CH_2-CH_2-$ oder $-CH=CH-$Gruppe darstellt.

0161604

Die Reduktion wird vorzugsweise mit einem Metallhydrid wie Lithiumaluminiumhydrid oder Diboran oder mit einem Komplex aus Boran und einem Thioäther, z.B. mit Boran-Dimethylsulfid-Komplex, in einem geeigneten Lösungsmittel wie Diäthyläther oder Tetrahydrofuran bei Temperaturen zwischen 0 und 50°C, vorzugsweise jedoch bei Temperaturen zwischen 10 und 25°C, durchgeführt.

f) Zur Herstellung von Verbindungen der allgemeinen Formel I, in der A die -COCO-Gruppe darstellt:

Oxidation einer Verbindung der allgemeinen Formel

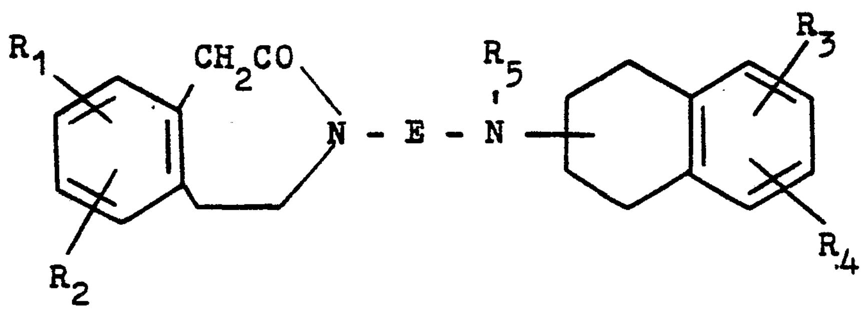

,(VIII)

in der

$R_1$ bis $R_5$ und E wie eingangs definiert sind.

Die Oxidation wird vorzugsweise mit einem Oxidationsmittel wie Kaliumpermanganat, Selendioxid oder Natriumdichromat in einem geeigneten Lösungsmittel oder Lösungsmittelgemisch wie Wasser, Wasser/Dioxan, Eisessig, Wasser/Essigsäure oder Acetanhydrid bei Temperaturen zwischen 0 und 100°C, vorzugsweise bei Temperaturen zwischen 20 und 80°C, durchgeführt.

g) Zur Herstellung von Verbindungen der allgemeinen Formel I, in der A die -NH-CO-Gruppe und E eine gegebenenfalls durch eine Alkylgruppe mit 1 bis 3 Kohlenstoffatomen substituierte Alkylengruppe mit 2 bis 4 Kohlenstoffatomen darstellen:

Umsetzung einer Verbindung der allgemeinen Formel

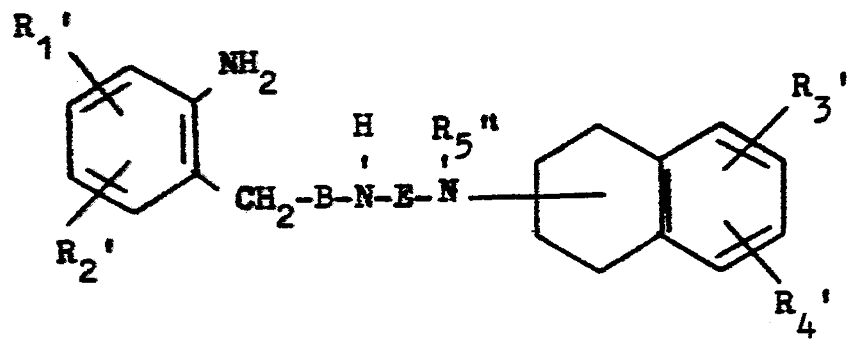

,(IX)

in der

B und E wie eingangs definiert sind,

$R_1'$ eine durch eine Schutzgruppe geschützte Hydroxy-, Amino- oder Alkylaminogruppe darstellt oder die für $R_1$ eingangs erwähnten Bedeutungen besitzt,

$R_2'$ eine durch eine Schutzgruppe geschützte Hydroxygruppe darstellt oder die für $R_2$ eingangs erwähnten Bedeutungen besitzt,

$R_3'$ und $R_4'$, die gleich oder verschieden sein können, jeweils eine durch eine Schutzgruppe geschützte Hydroxy-, Amino- oder Alkylaminogruppe darstellen oder die für $R_3$ bzw. $R_4$ eingangs erwähnten Bedeutungen besitzen,

$R_5''$ eine Schutzgruppe für eine Aminogruppe darstellt oder mit Ausnahme von Wasserstoff die für $R_5$ eingangs erwähnten Bedeutungen besitzt, mit einem Kohlensäurederivat der allgemeinen Formel

$$W - CO - W \quad ,(X)$$

in der

W, die gleich oder verschieden sein können, jeweils eine nukleophile Austrittsgruppe, z.B. ein Chlor- oder Bromatom, eine Alkoxygruppe mit 1 bis 3 Kohlenstoffatomen, eine Imidazolyl-(1)-gruppe oder auch eine Trichlormethoxygruppe, wenn der andere der Reste W ein Chlor- oder Bromatom darstellt, bedeuten, und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und
als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Essigester, Methylenchlorid, Tetrachlorkohlenstoff, Benzol, Tetrahydrofuran, Benzol/Tetrahydrofuran, Dioxan oder Acetonitril zweckmäßigerweise bei Temperaturen zwischen 0 und 150°C, vorzugsweise jedoch bei der Siedetemperatur des verwendeten Lösungsmittels, z.B. bei Temperaturen zwischen 40 und 100°C, und gegebenenfalls in Gegenwart eines säurebindenden Mittels wie Kaliumcarbonat, Natriumhydroxid, Kaliumhydroxid, Pyridin oder Triethylamin, wobei die letzteren gleichzeitig auch als Lösungsmittel dienen können, durchgeführt. Die Umsetzung kann jedoch auch ohne Lösungsmittel durchgeführt werden. Bedeutet in einer eingesetzten Verbindung der allgemeinen Formel X mindestens einer der Reste W eine Alkoxygruppe mit 1 bis 3 Kohlenstoffatomen, so wird die Umsetzung vorzugsweise in einem Überschuß des eingesetzten Esters als Lösungsmittel durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen ziwschen 0 und 100°C, vorzugsweise bei der Siedetemperatur des Reaktionsgemisches. Die Abspaltung eines Benzylrestes kann jedoch auch hydrogenolytisch erfolgen, z.B. mit Wasserstoff in Gegenwart eines Katalysators wie Palladium/Kohle, in einem Lösungsmittel wie Methanol, Ethanol, Essisäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

h) Umsetzung einer Verbindung der allgemeinen Formel

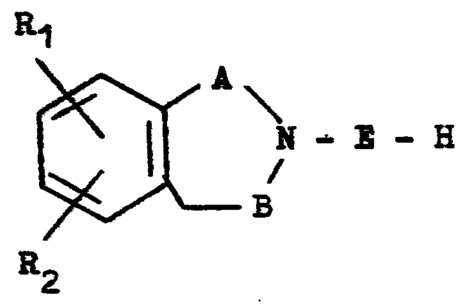

,(XI)

in der
A, B, E, $R_1$ und $R_2$ wie eingangs definiert sind, wobei jedoch im Rest E zwei Wasserstoffatome in einer $-CH_2-$ oder $CH_3$-Gruppe des Restes E durch ein Sauerstoffatom ersetzt sind, mit einer Verbindung der allgemeinen Formel

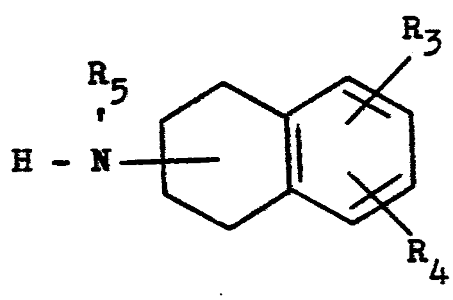

,(XII)

in der
$R_3$ bis $R_5$ wie eingangs definiert sind, in Gegenwart eines Reduktionsmittels.

Die Umsetzung wird zweckmäßigerweise in einem geeigneten Lösungsmittel oder Lösungsmittelgemisch wie Methanol, Ethanol, Ethanol/Essigsäureethylester oder Dioxan bei Temperaturen zwischen 0 und 100°C, vorzugsweise jedoch bei Temperaturen zwischen 20 und 80°C, durchgeführt.

Besonders vorteilhaft wird die reduktive Aminierung in Gegenwart eines komplexen Metallhydrids wie Lithium- oder Natriumcyanborhydrid vorzugsweise bei einem pH-Wert von 6-7 und bei Raumtemperatur oder zur Herstellung von Verbindungen der allgemeinen Formel I, in der $R_5$ ein Wasserstoffatom darstellt, in Gegenwart von Palladium/Kohle bei einem Wasserstoffdruck von 5 bar, durchgeführt. Hierbei können gegebenenfalls vorhandene Benzylgruppen gleichzeitig hydrogenolytisch abgespalten und/oder Doppelbindungen aufhydriert werden.

Die erhaltenen Verbindungen der allgemeinen Formel I lassen sich ferner in ihre Säureadditionssalze, insbesondere in ihre physiologisch verträgliche Säureadditionssalze mit anorganischen oder organischen Säuren überführen. Als Säuren kommen hierbei beispielsweise Salzäure, Bromwasserstoffsäure, Schwefelsäure, Phosphorsäure, Essigsäure, Milchsäure, Zitronensäure, Weinsäure, Bernsteinsäure, Maleinsäure oder Fumarsäure in Betracht.

Die als Ausganssstoffe verwendeten Verbindungen der allgemeinen Formeln II bis XII sind teilweise literaturbekannt bzw. man erhält sie nach an sich bekannten Verfahren.

So erhält man beispielsweise eine Ausgangsverbindung der allgemeinen Formel II durch Umsetzung eines entsprechenden Benzazepins mit einer entsprechenden Halogenverbindung und gegebenenfalls durch anschließende Umsetzung mit einem entsprechenden Amin. Das hierfür erforderliche in 3-Stellung unsubstituierte entsprechende Benzazepin erhält man durch Cyclisierung einer entsprechenden Verbindung, z.B. durch Cyclisierung einer Verbindung der allgemeinen Formel

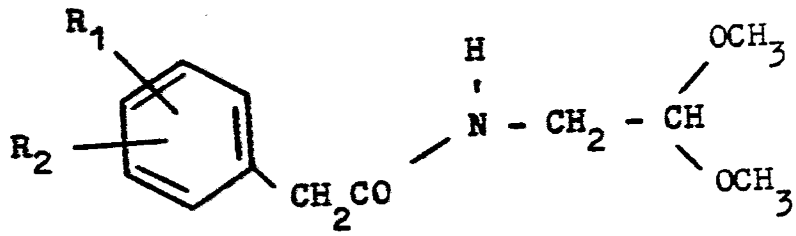

,(XIII)

oder auch der allgemeinen Formel

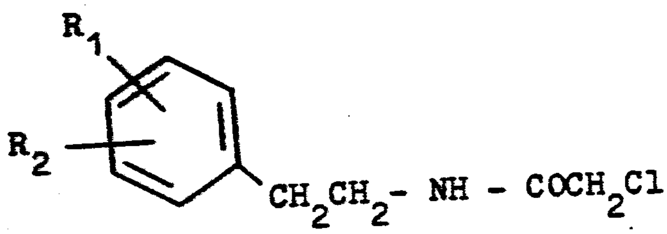

,(XIV)

gegebenenfalls anschließender katalytischer Hydrierung und/oder Reduktion der Carbonylgruppe beispielsweise mit Natriumborhydrid/Eisessig (siehe EP-Al 0.007.070) und/oder Oxidation, z.B. mit Selendioxid.

Eine als Ausgangsstoff verwendete Verbindung der allgemeinen Formeln IV bis VIII erhält man vorzugsweise durch Umsetzung einer entsprechenden Halogenverbindung mit einem entsprechenden Amin und gegebenenfalls anschließende Abspaltung von Schutzresten, die zum Schutz von Hydroxy- und/oder Aminogruppen verwendet werden.

Eine als Ausgangsstoff verwendete Verbindung der allgemeinen Formel IX erhält man beispielsweise durch Reduktion einer entsprechenden Nitroverbindung.

Eine Verbindung der allgemeinen Formeln III oder XII erhält man beispielsweise durch Umsetzung eines entsprechenden Tetralons mit einem entsprechenden Amin und anschließender Reduktion.

Eine Verbindung der allgemeinen Formel XI erhält man beispielsweise durch Umsetzung eines in 3-Stellung unsubstituierten entsprechenden Benzazepins mit einem entsprechenden Halogenacetal bzw. Halogenketal und anschließende Hydrolyse.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere bei geringen zentralen Nebenwirkungen, eine lang anhaltende herzfrequenzsenkende Wirkung sowie eine Herabsetzung des $O_2$-Bedarfs des Herzens auf.

Beispielsweise wurden die Verbindungen

A = 1-(7,8-Dimethoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan-hydrochlorid,

B = 1-(7,8-Dimethoxy-1,3,4,5-tetrahydro-2H-3-benzazepin-2-on-3-yl)-3-[N-(6,7-dimethoxy-1,2,3,4-tetrahydronaphthyl-2)-amino]-propan-hydrochlorid,

C = 1-(7,8-Dimethoxy-1,3,4,5-tetrahydro-2H-3-benzazepin-2-on-3-yl)-3-[N-methyl-N-(7-methoxy-1,2,3,4-tetrahydronaphthyl-2)-amino]-propan-hydrochlorid und

D = 1-(7,8-Dimethoxy-1,3,4,5-tetrahydro-2H-3-benzazepin-2-on-3-yl)-3-[N-methyl-N-(6-methoxy-1,2,3,4-tetrahydronaphthyl-2)-amino]-propan-hydrochlorid

auf ihre biologischen Eigenschaften wie folgt untersucht:

Wirkung auf die Herzfrequenz an Ratten:

Die Wirkung der zu untersuchenden Substanzen auf die Herzfrequenz wurde pro Dosis an 2 Ratten mit einem durchschnittlichen Gewicht von 250-300 g untersucht. Hierzu wurden die Ratten mit Pentobarbital (50 mg/kg i.p. und 20 mg/kg s.c.) narkotisiert. Die zu untersuchenden Substanzen wurden in wäßriger Lösung in die Vena jugularis injiziert (0,1 ml/ 100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minuten (S/min).

Die nachfolgende Tabelle enthält die gefundenen Werte:

| Substanz | Dosis [mg/kg] | Herzfrequenzsenkung, gemessen 20 Minuten nach Substanzapplikation [S/min] |
|---|---|---|
| A | 5,0 | - 176 |
| B | 5,0 | - 203 |
| C | 5,0 | - 184 |
| D | 5,0 | - 165 |

Wirkung auf die Herzfrequenz an Katzen:

Die Wirkung der zu untersuchenden Substanzen auf die Herzfrequenz wurde pro Dosis an 7 Katzen beiderlei Geschlechts mit einem durchschnittlichen Gewicht von 2,5 - 3,5 kg untersucht. Hierzu wurden die Katzen mit Chloralose (80 mg/kg) narkotisiert. Die zu untersuchende Substanz wurde in wäßriger Lösung in die Vene saphena injiziert.

Die Herzfrequenz wurde vor und nach Substanzgabe mit Hilfe eines Grass-Tachographen aus dem Elektrocardiogramm (Brustwandableitung) auf einem Grass-Polygraphen registriert:

Die nachfolgende Tabelle enthält die gefundenen Werte:

| Substanz | Dosis mg/kg | Herzfrequenzsenkung | Halbwertzeit (Minuten) |
|---|---|---|---|
| A | 0,3 i.v. | - 30 % | > 120 |
| A | 1,0 i.v. | - 58 % | > 120 |
| B | 1,0 i.v. | - 55 % | > 120 |

Die erfindungsgemäß hergestellten Verbindungen weisen in therapeutischen Dosen keinerlei toxische Nebenwirkungen auf. So konnten beipielsweise bei einer intravenösen Applikation der Substanz A und B auch in einer hohen Dosis von 20 mg/kg an Mäusen, außer einer geringen Sedation keine toxischen Nebenwirkungen beobachtet werden.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderlichen Dosierung beträgt zweckmäßigerweise ein- bis zweimal täglich 0,03 bis 0,4 mg/kg Körpergewicht, vorzugsweise 0,07 bis 0,25 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/ Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

Herstellung der Ausgangsverbindungen:

Beispiel A

7,8-Dimethoxy-1,3-dihydro-2H-3-benzazepin-2-on

a) 3,4-Dimethoxy-phenylessigsäurechlorid

Zu einer Suspension von 549,4 g 3,4-Dimethoxy-phenylessigsäure in 600 ml Methylenchlorid werden während 2 Stunden 600 ml Thionylchlorid unter Rühren zugetropft. Nach beendeter Gasentwicklung (16 Stunden) wird noch eine Stunde am Rückfluß gekocht. Nach dem Entfernen der leicht flüchtigen Komponenten wird der Rückstand im Vakuum destilliert.
Ausbeute: 486 g (80,8 % der Theorie),
Kp: 134-136°C/1,95 mbar

b) N-(2,2-Dimethoxyethyl)-3,4-dimethoxy-phenylacetamid

Unter Eiskühlung wird eine Lösung von 485,2 g 3,4-Dimethoxy-phenylessigsäurechlorid in 1,1 l Methylenchlorid bei 15-20°C zu einer Lösung von 246,2 ml Aminoacetaldehyddimethylacetal und 315 ml Triäthylamin in 2,2 l Methylenchlorid zugetropft und eine Stunde bei 16-18°C nachgerührt. Anschließend wird mehrfach mit Wasser extrahiert, über Magnesiumsulfat getrocknet und eingedampft. Das erhaltende Öl kristallisiert langsam durch.
Ausbeute: 608 g (95 % der Theorie),
Schmelzpunkt: 66-69°C.

c) 7,8-Dimethoxy-1,3-dihydro-2H-3-benzazepin-2-on

Eine Lösung von 600,6 g N-(2,2-Dimethoxyethyl)-3,4-dimethoxy-phenylacetamid in 3 l konzentrierter Salzsäure wird mit 3 l Eisessig versetzt. Nach 17-stündigem Stehenlassen bei

Raumtemperatur wird der Ansatz auf Eis gegossen. Die ausgefallenen Kristalle werden abgesaugt, mit Wasser neutral gewaschen und getrocknet.
Ausbeute: 350 g (75,4 % der Theorie),
Schmelzpunkt: 234-237°C.

Beispiel B

7,8-Dimethoxy-1,3,4,5-tetrahydro-2H-3-benzazepin-2-on

Eine Suspension von 21,9 g (0,1 Mol) 7,8-Dimethoxy-1,3-dihydro-2H-3-benzazepin-2-on und 1,5 g Palladium/Kohle (10%ig) in 200 ml Eisessig wird bei 50°C und einem Wasserstoffdruck von 5 bar hydriert. Nach Abfiltrieren des Katalysators wird das Lösungsmittel im Vakuum eingedampft und der Rückstand in Methylenchlorid aufgenommen. Nach Extraktion mit Natriumbicarbonat-Lösung und Waschen mit Wasser wird über Magnesiumsulfat getrocknet, eingeengt und über Kieselgel mit Methylenchlorid und anschließend mit steigenden Anteilen von Methanol (bis 10 % ) gereinigt.
Ausbeute: 12,6 g (57 % der Theorie),
Schmelzpunkt: 188-191°C.

Beispiel C

7,8-Dimethoxy-2,3,4,5-tetrahydro-1H-3-benzazepin

Zu einer Suspension von 1,3 g (6 mMol) 7,8-Dimethoxy-1,3,4,5-tetrahydro-2H-3-benzazepin-2-on und 1,1 g (3 mMol) Natriumborhydrid in 20 ml Dioxan wird eine Lösung von 1,8 g Eisessig in 10 ml Dioxan getropft, 3 Stunden unter Rückfluß gekocht, eingeengt und mit Wasser zersetzt. Das Gemisch wird 2 mal mit Methylenchlorid ausgeschüttelt, der Extrakt eingeengt und der Rückstand in Ether aufgenommen. Nach Filtration wird der Ether im Vakuum entfernt.
Ausbeute : 1,1 g (92,7 % der Theorie),
Schmelzpunkt: 86-89°C.

Beispiel D

6,9-Dimethoxy-1,3-dihydro-2H-3-benzazepin-2-on

2,0 g (0,007 Mol) N-(2,2-Dimethoxyethyl)-2,5-dimethoxyphenyl-acetamid werden mit 3 ml Polyphosphorsäure übergossen und 60 Minuten bei 90°C gerührt. Anschließend wird mit Eiswasser versetzt, das ausgefallene Produkt abgesaugt und getrocknet.
Ausbeute: 0,98 g (64 % der Theorie),
Schmelzpunkt: 188-191°C

Beispiel E

7,8-Dimethyl-1,3-dihydro-2H-3-benzazepin-2-on

Hergestellt analog Beispiel D aus N-(2,2-Dimethoxyethyl)-3,4-dimethyl-phenylacetamid und Polyphosphorsäure.
Ausbeute: 40,1 % der Theorie,
Schmelzpunkt: 220-224°C.

Beispiel F

7,8-Dimethoxy-1,3,4,5-tetrahydro-2H-3-benzazepin-2,4-dion

a) 7,8-Dimethoxy-2-amino-4-brom-1H-3-benzazepin-hydrobromid

3,7 g (0,017 Mol) 3,4-Dimethoxy-o-phenylen-diacetonitril werden in 10 ml Eisessig suspendiert und bei 20°C mit 12 ml 30%iger Bromwasserstoffsäure in Eisessig versetzt. 3 Stunden wird bei Raumtemperatur nachgerührt, der ausgefallene Niederschlag abgesaugt, mit Eisessig und anschließend mit Aceton/Ether gewaschen und getrocknet.
Ausbeute: 5,3 g (82,8 % der Theorie),
Schmelzpunkt: 210-211°C (Zers.).

b) 7,8-Dimethoxy-1,3,4,5-tetrahydro-2H-3-benzazepin-2,4-dion

5,3 g (0,014 Mol) 7,8-Dimethoxy-2-amino-4-brom-1H-3-benzazepin-hydrobromid werden in 100 ml 85°C heißem Wasser gelöst, mit 1,3 g wasserfreiem Natriumacetat versetzt und eine Stunde auf 90°C erhitzt. Das Reaktionsgemisch wird abgekühlt, abgesaugt, mit kaltem Wasser nachgewaschen und getrocknet.
Ausbeute: 2,9 g (88 % der Theorie),
Schmelzpunkt: 235°C (Zers.).

Beispiel G

7,8-Dimethoxy-2,3-dihydro-1H-3-benzazepin

Eine siedende Suspension von 0,8 g Lithiumaluminiumhydrid in 100 ml absolutem Dioxan wird mit 2,2g (0,01 Mol) 7,8-Dimethoxy-1,3-dihydro-2H-3-benzazepin-2-on versetzt und anschließend 3 Stunden unter Rückfluß erhitzt. Unter Eiswasserkühlung wird mit 10%iger Ammoniumchlorid-Lösung versetzt und der gebildete Niederschlag abgesaugt. Das Filtrat wird im Vakuum auf ein Volumen von etwa 20 ml eingeengt, der ausgefallene weiße Niederschlag abgesaugt und mit wenig Dioxan nachgewaschen.
Ausbeute: 0,9 g (43,8 % der Theorie),
Schmelzpunkz: 162-163°C.

Beispiel H

1-(7,8-Dimethoxy-1,3,4,5-tetrahydro-2H-3-benzazepin-2-on-3-yl)-3-chlor-propan

a) 1-(7,8-Dimethoxy-1,3-dihydro-2H-3-benzazepin-2-on-3-yl)-3-chlor-propan

131,5 g (0,6 Mol) 7,8.Dimethoxy-1,3-dihydro-2H-3-benzaze-

0161604

pin-2-on werden in 900 ml Dimethylsulfoxid suspendiert und unter Rühren mit 80,8 g (0,72 Mol) Kalium-tert.butylat versetzt. Nach 10 Minuten wird die erhaltene Lösung unter Kühlung mit Eiswasser zu 77 ml (0,72 Mol) 1-Brom-3-chlorpropan in 300 ml Dimethylsulfoxid getropft. Nach einer Stunde gießt man auf Eiswasser. Nach kurzer Zeit beginnt die schmierige Fällung zu kristallisieren. Der Niederschlag wird abgesaugt, in Aceton gelöst, mit Wasser nochmals ausgefällt, abgesaugt und getrocknet.
Ausbeute: 155,5 g (87,3 % der Theorie),
Schmelzpunkt: 101-103°C

b) 1-(7,8-Dimethoxy-1,3,4,5-tetrahydro-2H-3-benzazepin-2-on-3-yl)-3-chlor-propan

59,2 g (0,2 Mol) 1-(7,8-Dimethoxy-1,3-dihydro-2H-3-benzazepin-2-on-3-yl)-3-chlor-propan werden in 500 ml Eisessig in Anwesenheit von 5 g 10%iger Palladium-Kohle 6 Stunden bei 50°C und 5 bar hydriert. Der Katalysator wird abgesaugt, der Eisessig im Vakuum abdestilliert und der Rückstand nach Zugabe von Wasser mit Kaliumcarbonat neutralisiert. Der Niederschlag wird abgesaugt, mit Wasser salzfrei gewaschen und getrocknet.
Ausbeute: 53 g (89 % der Theorie),
Schmelzpunkt: 85-86°C

Beispiel I

1-(7-Brom-8-methoxy-1,3,4,5-tetrahydro-2H-3-benzazepin-2-on-3-yl)-3-chlor-propan

a) 8-Methoxy-1,3,4,5-tetrahydro-2H-3-benzazepin-2-on

56,8 g (0,3 Mol) 8-Methoxy-1,3-dihydro-2H-3-benzazepin-2-on (Schmelzpunkt: 190-191°C), gelöst in 600 ml Eisessig, werden

in Anwesenheit von 5 g 10%iger Palladiumkohle bei 80°C und 5 bar 12 Stunden hydriert. Der Katalysator wird abgesaugt und die Essigsäure im Vakuum abdestilliert. Der Rückstand wird mit Wasser versetzt, mit Kaliumcarbonat neutralisiert, der ausgefallene Niederschlag abgesaugt, mit Wasser gewaschen und getrocknet.
Ausbeute: 51,1 g (89,1 % der Theorie),
Schmelzpunkt: 160-161°C.

b) 7-Brom- und 9-Brom-8-methoxy-1,3,4,5-tetrahydro-2H-3-benzazepin-2-on

Zu 7,4 g (0,04 Mol) 8-Methoxy-1,3,4,5-tetrahydro-2H-3-benzazepin-2-on in 100 ml 80%iger Essigsäure werden bei 3-5°C unter Rühren 6,4 g = 2,03 ml (0,04 Mol) Brom in 10 ml Eisessig getropft. Nach 15 Minuten wird auf Eiswasser gegossen, mit Kaliumcarbonat neutralisiert, der Niederschlag abgesaugt, mit wenig Wasser gewaschen und getrocknet. Das erhaltene Isomerengemisch wird über eine Kieselgelsäule chromatographisch getrennt (Elutionsmittel: Essigester).
Ausbeute: 5,7 g (52,8 % der Theorie) 9-Brom-Isomeres
IR-Spektrum (Methylenchlorid): 3400 $cm^{-1}$ (NH)
1660 $cm^{-1}$ (C=O)
4,1 g (39 % der Theorie) 7-Brom-Isomeres
IR-Spektrum (Kaliumbromid): 3220 $cm^{-1}$ (NH)
1665 $cm^{-1}$ (CO)

c) 1-(7-Brom-8-methoxy-1,3,4,5-tetrahydro-2H-3-benzazepin-2-on-3-yl)-3-chlor-propan

Zu 1,35 g (5 mMol) 7-Brom-8-methoxy-1,3,4,5-tetrahydro-2H-3-benzazepin-2-on in 15 ml Dimethylsulfoxid werden 0,24 g (5,5 mMol) Natriumhydrid-Dispersion in Öl (55%ig) zugesetzt und 1/2 Stunde bei Raumtemperatur und 10 Minuten bei 35-40°C gerührt. Die Lösung wird zu 0,79 g (5,5 mMol) 1-Brom-3-chlorpropan in 5 ml Dimethylsulfoxid unter Rühren getropft.

Anschließend rührt man 2 Stunden bei Raumtemperatur, gießt auf Eiswasser und extrahiert 4 mal mit Methylenchlorid. Die Methylenchlorid-Extrakte werden mehrmals mit Wasser gewaschen, getrocknet und im Vakuum eingeengt. Der Rückstand wird über eine Kieselgelsäule mit Essigester als Elutionsmittel gereinigt.
Ausbeute: 210 mg (12 % der Theorie),
Schmelzpunkt: 119-120°C.

Beispiel J

1-(7-Methoxy-1,3,4,5-tetrahydro-2H-3-benzazepin-2-on-3-yl)-3-chlor-propan

a) 7-Methoxy-1,3,4,5-tetrahydro-2H-3-benzazepin-2-on

3,1 g (0,0136 Mol) N-Chloracetyl-N-(2-(3-methoxy-phenyl)-ethyl)-amin werden in 270 ml Ethanol und 1530 ml Wasser gelöst und 10 Stunden unter Stickstoffatmosphäre bei 20-25°C mit einer Quecksilber-Hochdrucklampe belichtet. Die Lösung wird auf ein Volumen von ca. 400 ml eingeengt, mit Natriumbicarbonat versetzt und mehrmals mit Essigester ausgeschüttelt. Die Extrakte werden über Magnesiumsulfat getrocknet, eingeengt und der Rückstand über eine Kieselgelsäule mit Essigester als Elutionsmittel gereinigt.
Ausbeute: 820 mg (31,5 % der Theorie),
Schmelzpunkt: 152-154°C.

b) 1-(7-Methoxy-1,3,4,5-tetrahydro-2H-3-benzazepin-2-on-3-yl)-3-chlor-propan

1,15 g (6 mMol) 7-Methoxy-1,3,4,5-tetrahydro-2H-3-benzazepin-2-on werden in 30 ml absolutem Tetramethylharnstoff gelöst, mit 300 mg 55%iger Natriumhydrid-Dispersion (in Öl) versetzt und unter einer Stickstoff-Atmosphäre 2 Stunden bei 20-25°C gerührt. Das erhaltene Reaktionsgemisch wird unter

Rühren bei 15-20°C unter Stickstoff zu 1,6 g (7,8 mMol) 1-chlor-3-jodpropan, gelöst in 20 ml Tetramethylharnstoff, getropft und 3 Stunden bei Raumtemperatur gerührt. Anschließend wird mit ca. 300 ml Essigester versetzt und 6 mal mit Wasser extrahiert. Die organische Lösung wird über Magnesiumsulfat getrocknet, eingeengt und der Rückstand über eine Kieselgelsäule mit Methylenchlorid und steigenden Anteilen Ethanol (bis 2 %) gereinigt.
Ausbeute: 410 mg (25,5 % der Theorie),
IR-Spektrum (Methylenchlorid): 1650 $cm^{-1}$ (CO).

Beispiel K

1-(7-Nitro-8-methoxy-1,3,4,5-tetrahydro-2H-3-benzazepin-2-on-3-yl)-3-chlor-propan

28,5 g (0,106 Mol) 1-(8-Methoxy-1,3,4,5-tetrahydro-2H-3-benzazepin-2-on-3-yl)-3-chlor-propan werden in 350 ml konzentrierter Salpetersäure 1/2 Stunde bei 20-25°C gerührt. Die Lösung wird auf Eiswasser gegosen, mit Kaliumcarbonat neutralisiert und 2 mal mit Methylenchlorid extrahiert. Der Extrakt wird über Magnesiumsulfat getrocknet, im Vakuum eingeengt und der Rückstand über eine Kieselgelsäule mit Essigester als Elutionsmittel gereinigt.
Ausbeute: 11 g (33,2 % der Theorie),
Schmelzpunkt: 127-128°C.

Beispiel L

2-Methylamino-6,7-dimethoxy-1,2,3,4-tetrahydronaphthalin

a) 6,7-Dimethoxy-1,2,3,4-tetrahydronaphthalinon-(2) x Natriumhydrogensulfit

49,2 g (0,229 Mol) 3,4-Dimethoxyphenylessigsäurechlorid

werden in 500 ml Methylenchlorid gelöst, zu einer Suspension von 123 g (0,923 Mol) Aluminiumchlorid in 3800 ml Methylenchlorid bei -5°C getropft. Nach einstündigem Einleiten von Ethylen wird der Ansatz mit Eiswasser zersetzt, mit 2n Salzsäure und mit gesättigter Natriumhydrogencarbonatlösung extrahiert. Die organische Phase wird über Magnesiumsulfat getrocknet, eingedampft und mit gesättigter Natriumhydrogensulfit-Lösung versetzt.
Ausbeute: 28,2 g (39,7 % der Theorie),
Schmelzpunkt: ab 160°C (Zers.).

b) <u>2-Methylamino-6,7-dimethoxy-1,2,3,4-tetrahydronaphthalin-hydrochlorid</u>

92,8 g (0,45 Mol) 6,7-Dimethoxy-1,2,3,4-tetrahydronaphthalinon-2 werden unter Stickstoffbegasung als teilweise Suspension in 1125 ml absolutem Ethanol mit 77,1 ml (1.35 Mol) Essigsäure und 112,5 g Molekularsieb 3A versetzt. Nach Einleitung von 42,5 g (1,35 Mol) Methylamin bei Raumtemperatur wird die Lösung von Molekularsieb abdekantiert, 4,65 g Platin (IV)-oxid zugegeben und 40 Minuten bei Raumtemperatur und einem Wasserstoffdruck von 5 bar hydriert. Nach Abfiltrieren des Katalysators wird das Lösungsmittel im Vakuum bis auf ein Volumen von 500 ml eingedampft und mit etherischer Salzsäure das Hydrochlorid gefällt.
Ausbeute: 75,2 g (64,8 % der Theorie),
Schmelzpunkt: 256-258°C.

<u>Beispiel M</u>

<u>2-Benzylamino-6,7-dimethoxy-1,2,3,4-tetrahydro-naphthalin-hydrochlorid</u>

Hergestellt analog Beispiel L durch Umsetzung von 6,7-Di-

0161604

methoxy-1,2,3,4-tetrahydronaphthalinon-2 mit Benzylamin.
Ausbeute: 75,4 % der Theorie,
Schmelzpunkt: 237-238°C.

## Beispiel N

5,6-Dimethoxy-2-methylamino-1,2,3,4-tetrahydronaphthalin-hydrochlorid

a) 5,6-Dimethoxy-1,2,3,4-tetrahydronaphthalin-on-(2 x Natriumhydrogensulfit

Zu 12 g (0,055 Mol) 2,5,6-Trimethoxynaphthalin in 220 ml absolutem Ethanol werden unter Rühren innerhalb 45 Minuten portionsweise 16,4 g (0,715 Mol) Natrium gegeben. Zur Vervollständigung der Reaktion wird anschließend noch 1 Stunde unter Rückfluß gekocht. Nach Abkühlung im Eisbad wird das ausgefallene Natriumchlorid abgesaugt und mit Methylenchlorid nachgewaschen. Das Filtrat wird nach Zugabe von weiterem Methylenchlorid kurz verrührt, die organische Phase abgetrennt und die wässrig-saure Lösung nochmals mit Methylenchlorid ausgeschüttelt. Die organischen Lösungen werden vereinigt, 1 mal mit Wasser gewaschen, über Magnesiumsulfat getrocknet und eingedampft. Der Rückstand wird mit einer Lösung von 25 g Natriumhydrogensulfit in 80 ml Wasser und 20 ml Ethanol versetzt.
Ausbeute: 12,5 g (73,2 % der Theorie).

b) 5,6-Dimethoxy-2-methylamino-1,2,3,4-tetrahydronaphthalin-hydrochlorid

Hergestellt analog Beispiel L.
Ausbeute: 45,6 % der Theorie,
Schmelzpunkt: 203-204°C.

Analog Beispiel N wurden folgende Verbindungen hergestellt:

2-Methylamino-1,2,3,4-tetrahydronaphthalin-hydrochlorid
Ausbeute: 69,8 % der Theorie,
Schmelzpunkt: 180-181°C

5-Methoxy-2-methylamino-1,2,3,4-tetrahydro-naphthalinhydrochlorid
Ausbeute: 20,6 % der Theorie,
Schmelzpunkt: 205-206°C

7-Methoxy-2-methylamino-1,2,3,4-tetrahydronaphthalin-hydrochlorid
Ausbeute: 48,7 % der Theorie,
Schmelzpunkt: 216-219°C

6-Methoxy-2-methylamino-1,2,3,4-tetrahydronaphthalin-hydrochlorid
Ausbeute: 50,6 % der Theorie,
Schmelzpunkt: 167-168°C

8-Methoxy-2-methylamino-1,2,3,4-tetrahydronaphthalin-hydrochlorid
Ausbeute: 41,5 % der Theorie,
Schmelzpunkt: 145-146°C

## Beispiel O

### 2-Amino-5-methoxy-1,2,3,4-tetrahydronaphthalin-hydrochlorid

17,6 g (0,1 Mol) 5-Methoxy-1,2,3,4-tetrahydronaphthalin-on-(2) werden in 800 ml Methanol und 200 ml 1,2-Dichloräthan gelöst, mit 77 g Ammoniumacetat und 5,4 g Natriumcyanborhydrid versetzt und 2 Tage bei Labortemperatur gerührt. Das

Reaktionsgemisch wird mit konzentrierter Salzsäure auf pH 2 eingestellt und das Lösungsmittel im Vakuum abdestilliert. Der Rückstand wird in Essigester/Wasser gelöst. Die wässrige Phase wird abgetrennt, unter Kühlung mit 50%iger Natronlauge alkalisch gestellt und 2 mal mit Methylenchlorid extrahiert. Der Extrakt wird getrocknet, mit Bleicherde/A-Kohle behandelt und eingedampft. Der Rückstand wird in Aceton gelöst und mit etherischer Salzsäure das Hydrochlorid gefällt.
Ausbeute: 2 g (94, % der Theorie),
Schmelzpunkt: 260-262°C (Zers.)

Beispiel P

3-(7,8-Dimethoxy-1,3-dihydro-2H-3-benzazepin-2-on-3-yl)-propionaldehyd

a) 3-(7,8-Dimethoxy-1,3-dihydro-2H-3-benzazepin-2-on-3-yl)-propionaldehyd-diethylacetal

Hergestellt analog Beispiel H/a durch Umsetzung von 7,8-Dimethoxy-1,3-dihydro-2H-3-benzazepin-2-on mit 3-Chlor-propionaldehyddiethylacetal.
Ausbeute: 93,3 % der Theorie.

b) 3-(7,8-Dimethoxy-1,3-dihydro-2H-3-benzazepin-2-on-3-yl)-propionaldehyd

3,5 g (0,1 Mol) 3-(7,8-Dimethoxy-1,3-dihydro-2H-3-benzazepin-2-on-3-yl)-propionaldehyd-diäthylacetal werden in 50 ml 2 n Schwefelsäure und 50 ml Ethanol 2 Stunden auf 40°C erwärmt. Im Vakuum wird der Alkohol abdestilliert, der Rückstand unter Kühlung mit gesättigter Kaliumcarbonat-Lösung alkalisch gestellt und mehrmals mit Essigester ausgeschüttelt. Der Essigester-Extrakt wird 2 mal mit 5%iger Natriumhydrogensulfit-Lösung ausgeschüttelt. Der Bisulfit-Extrakt wird mit konzentrierter Salzsäure angesäuert und zur Entfernung des Schwefeldioxids 1/2 Stunde auf 40°C im Vakuum erhitzt. Anschließend wird mit gesättigter Kaliumcarbonat-Lösung versetzt, mehrmals mit Methylenchlorid extrahiert; über Magnesiumsulfat getrocknet und eingedampft.
Schmelzpunkt: 95-96°C
Ausbeute: 1,7 g (61,8 % der Theorie),

Beispiel Q

1-Amino-3-[N-methyl-N-(7-methoxy-1,2,3,4-tetrahydronaphthyl-2)-amino]-propan

a) 2-[N-Methyl-N-(2-cyano-ethyl)-amino]-7-methoxy-1,2,3,4-tetrahydronaphthalin

5,76 g (0,0253 Mol) 7-Methoxy-2-methylamino-1,2,3,4-tetrahydro-naphthalin werden unter Rühren in 100 ml Methanol gelöst. 2,07 ml (0,0316 Mol) Acrylnitril werden zugegeben und das Gemisch wird 3 Stunden auf 50-55°C erhitzt. Anschließend wird das Lösungsmittel abdestilliert.
Ausbeute: 6,2 g (100 % der Theorie),
Rf-Wert: 0,75 (Aluminiumoxyd, Laufmittel: Methylenchlorid)

b) 1-Amino-3-[N-methyl-N-(7-methoxy-1,2,3,4-tetrahydronaphthyl-2)-amino]-propan

6,7 g (0,0274 Mol) 2-[N-Methyl-N-(2-cyano-ethyl)-amino]-7-methoxy-1,2,3,4-tetrahydronaphthalin werden in 80 ml bei 20°C mit Ammoniak gesättigtem Methanol gelöst, mit 0,8 g Raney-Nickel versetzt und bei 50°C und 5 bar Wasserstoffüberdruck 3 Stunden hydriert. Der Katalysator wird abfiltriert und das Filtrat einrotiert.

Ausbeute: 5,4 g (79,4 % der Theorie),
Rf-Wert: 0,25 (Aluminiumoxyd, Laufmittel: 95 Volumenteile Methylenchlorid + 5 Volumenteile Ethanol)

Analog Beispiel Q wird folgende Verbindung hergestellt:

1-Amino-3-[N-methyl-N-(6,7-dimethoxy-1,2,3,4-tetrahydronaphthyl-2)-amino]-propan
Ausbeute: 100 % der Theorie,
Rf-Werte: 0,27 (Aluminiumoxyd, Laufmittel: 95 Volumenteile Methylenchlorid + 5 Volumenteile Ethanol)

## Beispiel R

**1-[2-(2-Amino-4,5-dichlorphenyl)-ethylamino]-3-[N-methyl-N-(6,7-dimethoxy-1,2,3,4-tetrahydronaphthyl-2)-amino]-propan**

a) 3,4-Dichlorphenylessigsäure

65,1 g (0,35 Mol) 3,4-Dichlor-benzylcyanid werden in 700 ml 2 m Natronlauge 2 Stunden unter Rückfluß gekocht, anschließend mit Aktivkohle versetzt und filtriert. Das Filtrat wird unter Eiswasser-Kühlung mit konzentrierter Salzsäure angesäuert, der ausgefallene Niederschlag abgesaugt, mit Wasser neutral gewaschen und getrocknet.
Ausbeute: 71 g (99 % der Theorie),
Schmelzpunkt: 82-84°C

b) 4,5-Dichlor-2-nitro-phenylessigsäure

66,7 g (0,325 Mol) 3,4-Dichlorphenylessigsäure werden unter Rühren bei 5°C portionsweise in ein Gemisch von 600 ml rauchender Salpetersäure und 300 ml konzentrierter Salpetersäure eingetragen. Eine Stunde wird unter weiterer Kühlung

nachgerührt und während weiterer 1,5 Stunden läßt man die Lösung auf 20°C ansteigen. Das Reaktionsgemisch wird auf Eis gegossen, der ausgefallene Niederschlag abgesaugt, mit Eiswasser neutral gewaschen und getrocknet.
Ausbeute: 54,2 g (66,7 % der Theorie),
Schmelzpunkt: 119-120°C

c) 1-[2-(4,5-Dichlor-2-nitro-phenyl)-1-oxo-ethylamino]-3-[N-methyl-N-(6,7-dimethoxy-1,2,3,4-tetrahydronaphthyl-2)-amino]-propan

10 g (0,04 Mol) 3,4-Dichlor-2-nitro-phenylessigsäure werden in 150 ml absolutem Essigester suspendiert und unter Rühren bei Raumtemperatur mit 8,1 g (0,05 Mol) N,N'-Carbonyldiimidazol versetzt. Der anfänglich ausgefallene Niederschlag ist nach 3 Stunden aufgelöst. In diese Lösung wird eine Lösung von 11,1 g (0,04 Mol) 1-Amino-3-[N-methyl-N-(6,7-dimethoxy-1,2,3,4-tetrahydronaphthyl-2)-amino]-propan in 150 ml absolutem Essigester zugetropft. Das erhaltene Reaktionsgemisch wird 2 Stunden bei 20°C nachgerührt und über Nacht stehen gelassen. 2 mal wird mit 2 m Natronlauge und 1 mal mit Wasser ausgeschüttelt, über Magnesiumsulfat getrocknet und das Lösungsmittel im Vakuum abdestilliert. Der Rückstand wird säulenchromatographisch über 900 g Aluminiumoxid N (Aktivität II, Elutionsmittel: Methylenchlorid + 1 % Äthanol) gereinigt.
Ausbeute: 18,2 g (89,2 % der Theorie),
IR-Spektrum (Methylenchlorid): 1670 $cm^{-1}$CO

d) 1-[2-(2-Amino-4,5-dichlorphenyl)-1-oxo-ethylamino]-3-[N-methyl-N-(6,7-dimethoxy-1,2,3,4-tetrahydronaphthyl-2)-amino]-propan

15,8 g (0,031 Mol) 1-[2-(4,5-Dichlor-2-nitro-phenyl)-1-oxo-ethylamino]-3-[N-methyl-N-(6,7-dimethoxy-1,2,3,4-tetrahydronaphthyl-2)-amino]-propan werden in 300 ml Methanol gelöst und mit 4,65 ml 98%igem Hydrazinhydrat versetzt. Unter Rüh-

ren werden portionsweise 2 g Raney-Nickel zugegeben. Anschließend wird 2 Stunden bei Raumtemperatur gerührt, der Katalysator abgesaugt und das Filtrat im Vakuum eingeengt. Der viskose Rückstand wird säulenchromatographisch über 400 g Aluminiumoxid N (Aktivität II, Laufmittel: Methylenchlorid und steigenden Anteilen von Ethanol (bis 1 %)) gereinigt.

Ausbeute: 13,9 g (93,3 % der Theorie),

IR-Spektrum (Methylenchlorid): 1660 $cm^{-1}$ CO

1515 $cm^{-1}$ Amid-II

e) 1-[2-(2-Amino-4,5-dichlorphenyl)-ethylamino]-3-[N-methyl-N-(6,7-dimethoxy-1,2,3,4-tetrahydronaphthyl-2)-amino]-propan

4,8 g (0,01 Mol) 1-[2-(2-Amino-4,5-dichlorphenyl)-1-oxo-ethylamino]-3-[N-methyl-N-(6,7-dimethoxy-1,2,3,4-tetrahydro-naphthyl-2)-amino]-propan werden in 100 ml absolutem Tetrahydrofuran gelöst und mit 1,25 g (1,1 ml) Bortrifluorid-diäthyläther-Komplex versetzt. Anschließend gibt man zu der Reaktionsmischung unter Rühren, unter Stickstoff-Atmosphäre und unter Erhitzen auf Rückflußtemperatur 1,6 ml einer 10 molaren Toluollösung des Boran-Dimethylsulfid-Komplexes, welche mit 20 ml absolutem Tetrahydrofuran verdünnt ist, und kocht weitere 8 Stunden unter Rückfluß. Das Lösungsmittel wird im Vakuum abdestilliert, der Rückstand in 100 ml 6 molarer Salzsäure eine Stunde auf 80°C erwärmt, abgekühlt und 2 mal mit Essigester ausgeschüttelt. Die salzsaure Lösung wird anschließend unter Eiswasserkühlung mit 50%iger Natronlauge alkalisch gestellt und mehrmals mit Methylenchlorid ausgeschüttelt. Der Extrakt wird über Magnesiumsulfat getrocknet, im Vakuum eingeengt und der Rückstand säulenchromatographisch über 170 g Aluminiumoxid N (Aktivität II, Laufmittel: Methylenchlorid und steigenden Anteilen von Ethanol (bis 5 %)) gereinigt.

Ausbeute: 1 g (21,4 % der Theorie).

Beispiel S

1-[2-(2-Amino-4,5-dimethoxyphenyl)-ethylamino]-3-[N-methyl-N-(6,7-dimethoxy-1,2,3,4-tetrahydro-naphthyl-2)-amino]-propan

a) 4,5-Dimethoxy-2-nitro-phenylessigsäure

49,05 g (0,25 Mol) 3,4-Dimethoxyphenylessigsäure werden unter Kühlen bei 30°C portionsweise in 500 ml konzentrierte Salpetersäure eingetragen und 15 Minuten unter weiterer Kühlung nachgerührt. Das Reaktionsgemisch wird auf 1,5 l Eiswasser gegossen, der ausgefallene Niederschlag abgesaugt, mit Eiswasser neutral gewaschen und getrocknet.
Ausbeute: 56,4 g (93,4 % der Theorie),
Schmelzpunkt: 209-211°C

b) 1-[2-(4,5-Dimethoxy-2-nitro-phenyl)-1-oxo-ethylamino]-3-[N-methyl-N-(6,7-dimethoxy-1,2,3,4-tetrahydronaphthyl-2)-amino]-propan

Hergestellt aus 5,25 g (0,0217 Mol) 4,5-Dimethoxy-2-nitrophenylessigsäure und 6,04 g (0,0218 Mol) 1-Amino-3-[N-methyl-N-(6,7-dimethoxy-1,2,3,4-tetrahydronaphthyl-2)-amino]-propan analog Beispiel Rc.
Ausbeute: 8,6 g (79 % der Theorie),
Rf-Wert: 0,8 (Aluminiumoxyd, Laufmittel: 95 Volumenteile Methylenchlorid + 5 Volumenteile Ethanol).

c) 1-[2-(2-Amino-4,5-dimethoxyphenyl)-1-oxo-ethylamino]-3-[N-methyl-N-(6,7-dimethoxy-1,2,3,4-tetrahydronaphthyl-2)-amino]-propan

8,5 g (0,017 Mol) 1-[2-(4,5-Dimethoxy-2-nitro-phenyl)-1-oxo-ethylamino]-3-[N-methyl-N-(6,7-dimethoxy-1,2,3,4-tetrahydronaphthyl-2)-amino]-propan werden in 100 ml Methanol gelöst, mit 9,8 g 10%igem Palladium auf Kohle versetzt und bei 20°C und 5 bar Wasserstoffdruck eine Stunde hydriert. Der Katalysator wird abfiltriert und das Filtrat einrotiert. Ausbeute: 7,8 g (97,6 % der Theorie), Rf-Wert: 0,2 (Aluminiumoxyd, Laufmittel: 98 Volumenteile Methylenchlorid + 2 Volumenteile Ethanol)

d) 1-[2-(2-Amino-4,5-dimethoxy-phenyl)-ethylamino]-3-[N-methyl-N-(6,7-dimethoxy-1,2,3,4-tetrahydro-naphthyl-2)-amino]-propan

Hergestellt aus 7,8 g (0,0165 Mol) 1-[2-(2-Amino-4,5-dimethoxyphenyl)-1-oxo-ethylamino]-3-[N-methyl-N-(6,7-dimethoxy-1,2,3,4-tetrahydronaphthyl-2)-amino]-propan analog Beispiel Re.
Ausbeute: 2,7 g (35,8 % der Theorie),
Rf-Wert: 0,2 (Aluminiumoxyd, Laufmittel: 95 Volumenteile Methylenchlorid + 5 Volumenteile Ethanol).

Beispiel T

1-[2-(2-Amino-4,5-dimethoxyphenyl)-ethylamino]-3-[N-methyl-N-(7-methoxy-1,2,3,4-tetrahydro-naphthyl-2)-amino]-propan

a) 4,5-Dimethoxy-2-nitro-phenylessigsäure

Hergestellt aus 49,05 g (0,25 Mol) 3,4-Dimethoxyphenylessigsäure analog Beispiel Sa.
Ausbeute: 56,4 g (93,4 % der Theorie),
Schmelzpunkt: 209-211°C

b) 1-[2-(4,5-Dimethoxy-2-nitro-phenyl)-1-oxo-ethylamino]-3-[N-methyl-N-(7-methoxy-1,2,3,4-tetrayhdro-naphthyl-2)-amino]-propan

Hergestellt aus 5,25 g (0,0217 Mol) 4,5-Dimethoxy-2-nitro-phenylessigsäure und 5,4 g (0,0217 Mol) 1-Amino-3-[N-methyl-N-(7-methoxy-1,2,3,4-tetrahydronaphthyl-2)-amino]-propan analog Beispiel Rc.
Ausbeute: 8,2 g (80,4 % der Theorie),
Schmelzpunkt: 116-118°C

c) 1-[2-(2-Amino-4,5-dimethoxyphenyl)-1-oxo-ethylamino]-3-[N-methyl-N-(7-methoxy-1,2,3,4-tetrahydronaphthyl-2)-amino]-propan

Hergestellt aus 8,2 g (0,0174 Mol) 1-[2-(4,5-Dimethoxy-2-nitro-phenyl)-1-oxo-ethylamino]-3-[N-methyl-N-(7-methoxy-1,2,3,4-tetrahydro-naphthyl-2)-amino]-propan analog Beispiel Sc.
Ausbeute: 7,4 g (96,7 % der Theorie).
Rf-Wert: 0,2 (Aluminiumoxid, Laufmittel: 98 Volumenteile Methylenchlorid + 2 Volumenteile Ethanol)

d) 1-[2-(2-Amino-4,5-dimethoxy-phenyl)-ethylamino]-3-[N-methyl-N-(7-methoxy-1,2,3,4-tetrahydronaphthyl-2)-amino]-propan

Hergestellt aus 6,73 g (0,0153 Mol) 1-[2-(2-Amino-4,5-dimethoxyphenyl)-1-oxo-ethylamino]-3-[N-methyl-N-(7-methoxy-1,2,3,4-tetrahydronaphthyl-2)-amino]-propan analog Beispiel Re.
Ausbeute: 4,2 g (64,5 % der Theorie),
Rf-Wert: 0,2 (Aluminiumoxyd, Laufmittel: 95 Volumenteile Methylenchlorid + 5 Volumenteile Ethanol)

## Herstellung der Endprodukte:

### Beispiel 1

1-[7,8-Dimethoxy-1,3-dihydro-2H-3-benzazepin-2-on-3-yl]-3-[N-methyl-N-(6,7-dimethoxy-1,2,3,4-tetrahydronaphthyl-2)-amino]-propan-hydrochlorid

Ein Gemisch von 1,72 g (0,0078 Mol) 2-Methylamino-6,7-dimethoxy-1,2,3,4-tetrahydronaphthalin, 1,09 ml (0,0078 Mol) Triethylamin und 2,3 g (0,0078 Mol) 1-(7,8-dimethoxy-1,3-dihydro-2H-3-benzazepin-2-on-3-yl)-3-chlor-propan wird innerhalb von 1 Stunde stufenweise auf 90°C Reaktionstemperatur gebracht und 2 Stunden bei dieser Temperatur erhitzt. Die anfängliche Suspendion geht langsam in eine klare Lösung über und beginnt nach etwa 30 Minuten gallertartig auszufallen. Das abgekühlte Reaktionsgemisch wird in 0,5 molarer Natronlauge/Essigester gelöst, die organische Phase mit Wasser gewaschen, über Magnesiumsulfat getrocknet, im Vakuum eingedampft und über 300 g Aluminiumoxid neutral Aktivität II mit Methylenchlorid und anschließend mit steigenden Anteilen von Ethanol (bis 20 %) gereinigt). Aus einer Lösung in Aceton wird mit etherischer Salzsäure das Hydrochlorid gefällt.

Ausbeute: 1,39 g (34,6 % der Theorie),

Schmelzpunkt: > 125°C (Zers.).

### Beispiel 2

1-[7,8-Dimethoxy-1,3,4,5-tetrahydro-2H-3-benzazepin-2-on-3-yl]-3-[N-methyl-N-(6,7-dimethoxy-1,2,3,4-tetrahydronaphthyl-2)-amino]-propan-hydrochlorid

Hergestellt analog Beispiel 1 durch Umsetzung von 2-Methylamino-6,7-dimethoxy-1,2,3,4-tetrahydronaphthalin mit 1-(7,8-Dimethoxy-1,3,4,5-tetrahydro-2H-3-benzazepin-2-on-3-yl)-3-chlor-propan.
Ausbeute: 44,5 % der Theorie,
Schmelzpunkt: 236-238°C.

## Beispiel 3

**1-[7,8-Dimethoxy-1,3,4,5-tetrahydro-2H-3-benzazepin-2-on-3-yl]-3-[N-benzyl-N-(6,7-dimethoxy-1,2,3,4-tetrahydronaphthyl-2)-amino]-propan**

Hergestellt analog Beispiel 1 durch Umsetzung von 2-Benzyl-amino-6,7-dimethoxy-1,2,3,4-tetrahydronaphthalin mit 1-(7,8-Dimethoxy-1,3,4,5-tetrahydro-2H-3-benzazepin-2-on-3-yl)-3-chlor-propan
Ausbeute: 39,6 % der Theorie,
IR-Spektrum (Methylenchlorid): 1645 $cm^{-1}$ (CO)

## Beispiel 4

**1-[7,8-Dimethoxy-1,3,4,5-tetrahydro-2H-3-benzazepin-2-on 3-yl]-3-[N-(6,7-dimethoxy-1,2,3,4-tetrahydronaphthyl-2)-amino]-propan-hydrochlorid**

0,78 g (0,0014 Mol) 1-[7,8-Dimethoxy-1,3,4,5-tetrahydro-2H-3-benzazepin-2-on-3-yl]-3-[N-benzyl-N-(6,7-dimethoxy-1,2,3,4-tetrahydronaphthyl-2)-amino]-propan werden in 20 ml Eisessig in Anwesenheit von 0,1 g 10%iger Palladium-Kohle 3 Stunden bei Raumtemperatur und 5 bar hydriert. Der Katalysator wird abgesaugt, der Eisessig im Vakuum abdestilliert, der Rückstand in Methylenchlorid/gesättigter Kaliumcarbonat-Lösung aufgenommen, die organische Phase mit Wasser gewaschen, über Magnesiumsulfat getrocknet, erneut einrotiert und über 100 g

Aluminiumoxid neutral Aktivität II mit Methylenchlorid und anschließend mit steigenden Anteilen von Ethanol (bis 40 %) gereinigt. Aus einer Lösung in Aceton wird mit etherischer Salzsäure das Hydrochlorid gefällt.
Ausbeute: 0,45 g (63,4 % der Theorie),
Schmelzpunkt: 222-223°C

Beispiel 5

1-[7,8-Dimethoxy-1,3,4,5-tetrahydro-2H-3-benzazepin-2-on 3-yl]-3-[N-methyl-N-(1,2,3,4-tetrahydronaphthyl-2)-amino]-propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan, Triäthylamin und 2-Methylamino-1,2,3,4-tetrahydronaphthalin.
Ausbeute: 29,6 % der Theorie,
Schmelzpunkt: 140-142°C (Zers.)

Beispiel 6

1-[7,8-Dimethoxy-1,3,4,5-tetrahydro-2H-3-benzazepin-2-on-3-yl]-3-[N-methyl-N-(5-methoxy-1,2,3,4-tetrahydronaphthyl-2)-amino]-propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan, Triethylamin und 5-Methoxy-2-methylamino-1,2,3,4-tetrahydronaphthalin.
Ausbeute: 28,6 % der Theorie,
Schmelzpunkt: 149-150°C (Zers.)

Beispiel 7

1-[7,8-Dimethoxy-1,3,4,5-tetrahydro-2H-3-benzazepin-2-on-3-yl]-3-[N-methyl-N-(7-methoxy-1,2,3,4-tetrahydronaphthyl-2)-amino]-propan-hydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan, Triethylamin und 7-Methoxy-2-methylamino-1,2,3,4-tetrahydronaphthalin.
Ausbeute: 40,9 % der Theorie,
Schmelzpunkt: 189-190°C (Zers.).

Beispiel 8

1-[7,8-Dimethoxy-1,3,4,5-tetrahydro-2H-3-benzazepin-2-on-3-yl]-3-[N-methyl-N-(6-methoxy-1,2,3,4-tetrahydronaphthyl-2)-amino]-propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan, Triethylamin und 6-Methoxy-2-methylamino-1,2,3,4-tetrahydronaphthalin.
Ausbeute: 57,3 % der Theorie,
Schmelzpunkt: 122-123°C.

Beispiel 9

1-[7,8-Dimethoxy-1,3,4,5-tetrahydro-2H-3-benzazepin-2-on-3-yl]-3-[N-methyl-N-(8-methoxy-1,2,3,4-tetrahydronaphthyl-2)-amino]-propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan, Triethylamin und 8-Methoxy-2-methylamino-1,2,3,4-tetrahydronaphthalin.
Ausbeute: 35,4 % der Theorie,
Schmelzpunkt: 141-143°C.

Beispiel 10

1-[7,8-Dimethoxy-1,3,4,5-tetrahydro-2H-3-benzazepin-2-on-3-yl]-3-[N-methyl-N-(5,6-dimethoxy-1,2,3,4-tetrahydronaphthyl-2)-amino]-propan-hydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan, Triethylamin und 5,6-Dimethoxy-2-methylamino-1,2,3,4-tetrahydronaphthalin.
Ausbeute: 17,3 % der Theorie,
Schmelzpunkt: 216-217°C

Beispiel 11

1-[7,8-Dimethoxy-1,3,4,5-tetrahydro-2H-3-benzazepin-2-on-3-yl]-3-[N-(1,2,3,4-tetrahydronaphthyl-2)-amino]-propan-hydrochlorid

1,5 g (0,0054 Mol) 3-(7,8-Dimethoxy-1,3-dihydro-2H-3-

benzazepin-2-on-3-yl)-propionaldehyd und 0,8 g (0,0054 Mol) 2-Amino-1,2,3,4-tetrahydronaphthalin werden in 150 ml Äthanol in Anwesenheit von 0,5 g 10 %iger Palladiumkohle 15 Stunden bei 5 bar und 60°C hydriert. Der Katalysator wird abgesaugt, das Filtrat im Vakuum eingedampft und der Rückstand über Kieselgel säulenchromatographisch mit Methylenchlorid und steigenden Anteilen von Ethanol (bis 4 %) gereinigt. Die sauberen Fraktionen werden eingedampft und aus Aceton das Hydrochlorid gefällt.
Ausbeute: 1,35 g (56,3 % der Theorie),
Schmelzpunkt: 229-230°C.

Beispiel 12

1-[7,8-Dimethoxy-1,3,4,5-tetrahydro-2H-3-benzazepin-2-on-3-yl]-3-[N-(5-methoxy-1,2,3,4-tetrahydronaphthyl-2)-amino]-propan-hydrochlorid

Hergestellt analog Beispiel 11 aus 3-(7,8-Dimethoxy-1,3-dihydro-2H-3-benzazepin-2-on-3-yl)-propionaldehyd und 2-Amino-5-methoxy-1,2,3,4-tetrahydronaphthalin.
Ausbeute: 46,3 % der Theorie,
Schmelzpunkt: 190°C

Beispiel 13

1-[7,8-Dimethoxy-2,3,4,5-tetrahydro-1H-3-benzazepin-3-yl]-3-[N-methyl-N-(6,7-dimethoxy-1,2,3,4-tetrahydro-naphthyl-2)-amino]-propan

1,4 g (0,0027 Mol) 1-[7,8-Dimethoxy-1,3,4,5-tetrahydro-2H-3-benzazepin-2-on-3-yl]-3-[N-methyl-N-(6,7-dimethoxy-1,2,3,4-tetrahydro-naphthyl-2)-amino]-propan-hydrochlorid werden zu einer Suspension von 0,24 g (0,0062 Mol) Lithiumaluminiumhydrid in 50 ml Diethylether gegeben und 7 Stunden am Rückfluß

gekocht. Anschließend wird mit Wasser und 15%iger Natronlauge versetzt, im Vakuum eingeengt und über 50 g Kieselgel (32-63 µm) mit Methylenchlorid und steigenden Anteilen von Ethanol (bis 15 %) als Elutionsmittel gereinigt.
Ausbeute: 0,6 g (47,2 % der Theorie),
Schmelzpunkt: 92-93°C

Beispiel 14

1-[7,8-Dimethoxy-1,3,4,5-tetrahydro-2H-3-benzazepin-1,2-dion-3-yl]-3-[N-methyl-N-(6,7-dimethoxy-1,2,3,4-tetrahydro-naphthyl-2)-amino]-propan-hydrochlorid

1,64 g (0,0034 Mol) 1-[7,8-Dimethoxy-1,3,4,5-tetrahydro-2H-3-benzazepin-2-on-3-yl]-3-[N-methyl-N-(6,7-dimethoxy-1,2,3,4-tetrahydro-naphthyl-2)-amino]-propan werden bei 70°C zu einer Suspension von 0,41 g (0,0036 Mol) Selendioxid und 0,34 g Celite in 17 ml 1,4-Dioxan und 0,68 ml Wasser gegeben und 40 Stunden am Rückfluß gekocht. Nach dem Abkühlen wird abgesaugt, im Vakuum einrotiert und über 40 g Kieselgel (32-63 µm) mit Methylenchlorid und steigenden Anteilen von Ethanol (bis 40 %) gereinigt. Die sauberen Fraktionen werden eingedampft und aus Aceton das Hydrochlorid gefällt.
Ausbeute: 0,6 g (35 % der Theorie),
Schmelzpunkt: > 145°C (Zers.).

Beispiel 15

1-[7,8-Dimethoxy-1,3,4,5-tetrahydro-2H-3-benzazepin-2-thion-3-yl]-3-[N-methyl-N-(6,7-dimethoxy-1,2,3,4-tetrahydro-naphthyl-2)-amino]-propan

2,97 g (0,006 Mol) 1-[7,8-Dimethoxy-1,3,4,5-tetrahydro-2H-3-benzazepin-2-on-3-yl]-3-[N-methyl-N-(6,7-dimethoxy-1,2,3,4-tetrahydro-naphthyl-2)-amino]-propan und 1,21 g (0,003 Mol) 2,4-Bis-(4-methoxyphenyl)-1,3-dithia-2,4-diphosphetan-2,4-disulfid werden in 12 ml Toluol 2 Stunden unter Rückfluß erhitzt. Nach Einengung im Vakuum wird über 240 g neutrales Aluminiumoxid mit der Aktivität II-III mit Methylenchlorid und steigenden Anteilen von Ethanol (bis 2 %) gereinigt.
Ausbeute: 1,4 g (46,3 % der Theorie),
Schmelzpunkt: 70-75°C

Beispiel 16

1-[7,8-Dimethoxy-1,3,4,5-tetrahydro-2H-3-benzazepin-2-on-3-yl]-3-[N-methyl-N-(6,7-dimethoxy-1,2,3,4-tetrahydronaphthyl-2)-amino]-propan-hydrochlorid

1,6 g (0,0033 Mol) 1-[7,8-Dimethoxy-1,3-dihydro-2H-3-benzazepin-2-on-3-yl]-3-[N-methyl-N-(6,7-dimethoxy-1,2,3,4-tetrahydro-naphthyl-2)-amino]-propan werden in 20 ml Eisessig in Anwesenheit von 50 mg 10%iger Palladium-Kohle 6 Stunden bei 50°C und 5 bar hydriert. Der Katalysator wird abgesaugt, der Eisessig im Vakuum abdestilliert und der Rückstand nach Zugabe von Wasser und Kaliumcarbonat neutralisiert. Der Niederschlag wird abgesaugt, mit Wasser salzfrei gewaschen und getrocknet. Aus einer Lösung in Aceton wird mit etherischer Salzsäure das Hydrochlorid gefällt.
Ausbeute: 1,2 g (70 % der Theorie),
Schmelzpunkt: 236-238°C

Beispiel 17

1-[1-Hydroxy-7,8-dimethoxy-1,3,4,5-tetrahydro-2H-3-benzazepin-2-on-3-yl]-3-[N-methyl-N-(6,7-dimethoxy-1,2,3,4-tetrahydro-naphthyl-2)-amino]-propan-hydrochlorid

0,70 g (0,0014 Mol) 1-[7,8-Dimethoxy-1,3,4,5-tetrahydro-2H-3-benzazepin-1,2-dion-3-yl]-3-[N-methyl-N-(6,7-dimethoxy-1,2,3,4-tetrahydro-naphthyl-2)-amino]-propan werden in 21 ml eines Gemisches aus Methanol/Wasser = 95:5 gelöst, mit 0,060 g (0,0016 Mol) Natriumborhydrid versetzt und bei Raumtemperatur 20 Minuten gerührt. Danach wird mit Salzsäure (2 Mol/l) angesäuert, mit methanolischem Ammoniak alkalisch gestellt und mit Methylenchlorid extrahiert, über Magnesiumsulfat getrocknet, eingeengt und der Rückstand über eine Säule (50 g Aluminiumoxid N, Aktivität II, Laufmittel: Methylenchlorid und anschließend mit steigenden Anteilen von Ethanol (bis 3 %)) gereinigt. Der Rückstand wird in Aceton gelöst und mit etherischer Salzsäure versetzt. Der ausgefallene Niederschlag wird abgesaugt, mit Ether gewaschen und getrocknet.

Ausbeute: 0,18 g (23,9 % der Theorie),

Schmelzpunkt: 141-143°C.

Beispiel 18

1-[7,8-Dimethoxy-1,3,4,5-tetrahydro-2H-1,3-benzodiazepin-2-on-3-yl]-3-[N-methyl-N-(7-methoxy-1,2,3,4-tetrahydro-naphthyl-2)-amino]-propan

2,0 g (0,005 Mol) 1-[2-(2-Amino-4,5-dimethoxyphenyl)-ethylamino]-3-[N-methyl-N-(7-methoxy-1,2,3,4-tetrahydronaphthyl-2)-amino]propan und 1 g (0,006 Mol) N,N'-Carbonyldiimidazol werden 60 Minuten in 40 ml absolutem Essigester unter Rückfluß gekocht. Nach Extraktion mit gesättigter Kaliumcarbonatlösung und mit Wasser wird das im Vakuum eingeengte Produkt in Methylenchlorid gelöst und mit Petroläther ausgefällt.
Ausbeute: 1,2 g (55 % der Theorie),
Schmelzpunkt: 163-165°C

Beispiel 19

1-[7,8-Dichlor-1,3,4,5-tetrahydro-2H-1,3-benzodiazepin-2-on-3-yl]-3-[N-methyl-N-(6,7-dimethoxy-1,2,3,4-tetrahydronaphthyl-2)-amino]-propan

a) 1-[2-(2-Amino-4,5-dichlorphenyl)-N-carbonyl-imidazolyl-ethylamino]-3-[N-methyl-N-(6,7-dimethoxy-1,2,3,4-tetrahydronaphtyl-2)-amino]-propan

1 g (0,0021 Mol) 1-[2-(2-Amino-4,5-dichlorphenyl)-ethylamino]-3-[N-methyl-N-(6,7-dimethoxy-1,2,3,4-tetrahydronaphthyl-2)-amino]-propan werden in 50 ml absolutem Essigester gelöst, mit 0,42 g (0,0026 Mol) N,N'-Carbonyldiimidazol versetzt und unter Stickstoffatmosphären eine Stunde am Rückfluß gekocht. Das Gemisch wird abgekühlt, zwei Mal mit 2N Natronlauge und zwei Mal mit Wasser ausgeschüttelt, über Magnesiumsulfat getrocknet und im Vakuum eingeengt. Der Rückstand wird säulenchromatographisch (80 g Aluminiumoxid N, Aktivität II, Laufmittel: Methylenchlorid und steigenden Anteilen von Ethanol (bis 3 %)) gereinigt. Zur weiteren Reinigung wird das Produkt in Methylenchlorid gelöst und mit Petroläther gefällt.
Ausbeute: 480 mg (40,8 % der Theorie),
Schmelzpunkt: 178°C.

b) 1-[7,8-Dichlor-1,3,4,5-tetrahydro-2H-1,3-benzodiazepin-2-on-3-yl]-3-[N-methyl-N-(6,7-dimethoxy-1,2,3,4-tetrahydronaphtyl-2)-amino]-propan

0,3 g (0,61 Mol) 1-[2-(2-Amino-4,5-dichlorphenyl)-N-carbonyl-imidazolyl-ethylamino]-3-[N-methyl-N-(6,7-dimethoxy-1,2,3,4-tetrahydronaphthyl-2)-amino]-propan werden in 10 ml Tetramethylharnstoff und 10 ml Ethyldiisopropylamin 3 Stunden unter Rückfluß gekocht. Im Vakuum wird Ethyldiisopropylamin abdestilliert, der Rückstand in Essigester aufgenommen, mehrmals mit Wasser ausgeschüttelt, über Magnesiumsulfat getrocknet und im Vakuum eingeengt. Der Rückstand wird in wenig Methylenchlorid gelöst und mit Petrolether gefällt.
Ausbeute: 190 mg (63,3 % der Theorie),
Schmelzpunkt: 195-197°C.

Beispiel 20

1-[7,8-Dimethoxy-1,3,4,5-tetrahydro-2H-1,3-benzodiazepin-2-on-3-yl]-3-[N-methyl-N-(6,7-dimethoxy-1,2,3,4-tetrahydronaphthyl-2)-amino]-propan

Hergestellt aus 1-[2-(2-Amino-4,5-dimethoxy-phenyl)-ethylamino]-3-[N-methyl-N-(6,7-dimethoxy-1,2,3,4-tetrahydro-naphthyl-2)-amino]-propan und N,N'-Carbonyldiimidazol analog Beispiel 18.
Ausbeute: 63 % der Theorie,
Schmelzpunkt: 187-188°C

| | | | | | | |
|---|---|---|---|---|---|---|
| Ber.: | C | 67,06 | H | 7,71 | N | 8,69 |
| Gef.: | | 66,91 | | 7,83 | | 8,61 |

## Beispiel 21

1-[7,8-Dimethoxy-1,3-dihydro-2H-3-benzazepin-3-yl]-3-[N-methyl-N-(6,7-dimethoxy-1,2,3,4-tetrahydro-naphthyl-2)-amino]-propan

Hergestellt aus 1-[7,8-Dimethoxy-1,3-dihydro-2H-3-benzazepin-2-on-3-yl]-3-[N-methyl-N-(6,7-dimethoxy-1,2,3,4-tetrahydro-naphthyl-2)-amino]-propan und Lithiumaluminiumhydrid analog Beispiel 13.

Ausbeute: 59,9 % der Theorie,

Schmelzpunkt: 115-116°C

| | | | | | | |
|---|---|---|---|---|---|---|
| Ber.: | C | 72,07 | H | 8,21 | N | 6,00 |
| Gef.: | | 71,80 | | 8,10 | | 5,88 |

## Beispiel 22

1-[7,8-Dimethoxy-1,3,4,5-tetrahydro-2H-3-benzazepin-2-on-3-yl]-3-[N-allyl-N-(6,7-dimethoxy-1,2,3,4-tetrahydro-naphthyl-2)-amino]-propan-hydrochlorid

Hergestellt aus 1-[7,8-Dimethoxy-1,3,4,5-tetrahydro-2H-3-benzazepin-2-on-3-yl]-3-chlorpropan und 2-Allylamino-6,7-dimethoxy-1,2,3,4-tetrahydronaphthalin analog Beispiel 1.

Ausbeute: 26 % der Theorie,

Schmelzpunkt: 226-228°C

| | | | | | | | | |
|---|---|---|---|---|---|---|---|---|
| Ber.: | C | 66,10 | H | 7,58 | Cl | 6,50 | N | 5,14 |
| Gef.: | | 65,89 | | 7,44 | | 6,54 | | 5,25 |

Beispiel 23

1-[7,8-Ethylendioxy-1,3,4,5-tetrahydro-2H-3-benzazepin-2-on-3-yl]-3-[N-methyl-N-(6,7-dimethoxy-1,2,3,4-tetrahydro-naphthyl-2)-amino]-propan-hydrochlorid

Hergestellt aus 1-[7,8-Ethylendioxy-1,3,4,5-tetrahydro-2H-3-benzazepin-2-on-3-yl]-3-chlorpropan und 6,7-Dimethoxy-2-methylamino-1,2,3,4-tetrahydronaphthalin analog Beispiel 1.
Ausbeute: 32 % der Theorie,
Schmelzpunkt: 152-157°C

| | | | | | | | | |
|---|---|---|---|---|---|---|---|---|
| Ber.: | C | 65,04 | H | 7,21 | Cl | 6,86 | N | 5,42 |
| Gef.: | | 64,81 | | 7,15 | | 6,94 | | 5,40 |

Beispiel 24

1-[7,8-Methylendioxy-1,3,4,5-tetrahydro-2H-3-benzazepin-2-on-3-yl]-3-[N-methyl-N-(6,7-dimethoxy-1,2,3,4-tetrahydro-naphthyl-2)-amino]-propan-hydrochlorid

Hergestellt aus 1-[7,8-Methylendioxy-1,3,4,5-tetrahydro-2H-3-benzazepin-2-on-3-yl]-3-chlorpropan und 6,7-Dimethoxy-2-methylamino-1,2,3,4-tetrahydro-napthalin analog Beispiel 1.
Ausbeute: 42 % der Theorie,
Schmelzpunkt: 158°C (Zers.)

| | | | | | | | | |
|---|---|---|---|---|---|---|---|---|
| Ber.: | C | 64,67 | H | 7,01 | Cl | 7,05 | N | 5,57 |
| Gef.: | | 64,50 | | 7,16 | | 7,14 | | 5,91 |

Beispiel 25

1-[7,8-Dimethoxy-1,3,4,5-tetrahydro-2H-3-benzazepin-2-on-3-yl]-3-[N-methyl-N-(6,7-dichlor-1,2,3,4-tetrahydro-naphthyl-2)-amino]-propan-hydrochlorid

Hergestellt aus 1-[7,8-Dimethoxy-1,3,4,5-tetrahydro-2H-3-benzazepin-2-on-3-yl]-3-chlor-propan und 6,7-Dichlor-2-methylamino-1,2,3,4-tetrahydro-napthalin analog Beispiel 1.
Ausbeute: 8,8 % der Theorie,
Schmelzpunkt: 130°C

| | | | | | | | | |
|---|---|---|---|---|---|---|---|---|
| Ber.: | C | 59,15 | H | 6,30 | Cl | 20,15 | N | 5,31 |
| Gef.: | | 58,37 | | 6,56 | | 20,38 | | 5,28 |

Beispiel 26

1-[7,8-Dimethoxy-1,3,4,5-tetrahydro-2H-3-benzazepin-2-on-3-yl]-3-[N-methyl-N-(6,7-methylendioxy-1,2,3,4-tetrahydro-naphthyl-2)-amino]-propan-hydrochlorid

Hergestellt aus 1-[7,8-Dimethoxy-1,3,4,5-tetrahydro-2H-3-benzazepin-2-on-3-yl]-3-chlorpropan und 2-Methylamino-6,7-methylendioxy-1,2,3,4-tetrahydro-naphthalin analog Beispiel 1.
Ausbeute: 12 % der Theorie,
Schmelzpunkt: > 148°C (Zers.)

| | | | | | | | | |
|---|---|---|---|---|---|---|---|---|
| Ber.: | C | 64,47 | H | 7,01 | Cl | 7,05 | N | 5,57 |
| Gef.: | | 64,50 | | 7,39 | | 7,02 | | 5,22 |

Analog den vorstehenden Beispielen werden folgende Verbindungen erhalten:

1-[7,8-Methylendioxy-1,3,4,5-tetrahydro-2H-3-benzazepin-2-on-3-yl]-3-[N-methyl-N-(6,7-methylendioxy-1,2,3,4-tetrahydro-naphthyl-2)-amino]-propan

1-(7-Brom-8-methoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan

1-(7-Chlor-8-methoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan

1-(7-Nitro-8-methoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan

1-(7-Amino-8-methoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan

1-(7,8-Dimethoxy-1,3,4,5-tetrahydro-2H-1,3-benzodiazepin-2-on-3-yl)-3-[N-methyl-N-(6-methoxy-1,2,3,4-tetrahydronaphthyl-2)-amino]-propan

Beispiel I

Tabletten zu 10 mg 1-(7,8-Dimethoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan-hydrochlorid

Zusammensetzung:
1 Tablette enthält:

| | |
|---|---|
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45°C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg

Beispiel II

Dragées zu 5 mg 1-(7,8-Dimethoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan-hydrochlorid

1 Dragéekern enthält:

| | |
|---|---|
| Wirksubstanz | 5,0 mg |
| Maisstärke | 41,5 mg |

| | |
|---|---|
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 130 mg

Beispiel III

Amullen zu 5 mg 1-(7,8-Dimethoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan-hydrochlorid

1 Ampulle enthält:

| | | |
|---|---|---|
| Wirksubstanz | | 5,0 mg |
| Sorbit | | 50,0 mg |
| Wasser für Injektionszwecke | ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg 1-(7,8-Dimethoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan-hydrochlorid

1 Zäpfchen enthält:

| | |
|---|---|
| Wirksubstanz | 0,015 g |
| Hartfett (z.B. Witepsol H 19 und W 45) | 1,685 g |
| | 1,700 g |

Herstellungsverfahren:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Beispiel V

Tropfenlösung mit 10 mg 1-(7,8-Dimethoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan-hydrochlorid

100 ml Lösungen enthalten:

| | |
|---|---|
| Wirksubstanz | 0,2 g |
| Hydroxyäthylcellulose | 0,15 g |
| Weinsäure | 0,1 g |

| | | |
|---|---|---|
| Sorbitlösung 70 % Trockensubstanz | | 30,0 g |
| Glycerin | | 10,0 g |
| Benzoesäure | | 0,15 g |
| Dest.Wasser | ad | 100 ml |

Herstellungsverfahren:

Dest.Wasser wird auf 70°C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

0161604

## Patentansprüche

1. Aminotetralinderivate der allgemeinen Formel

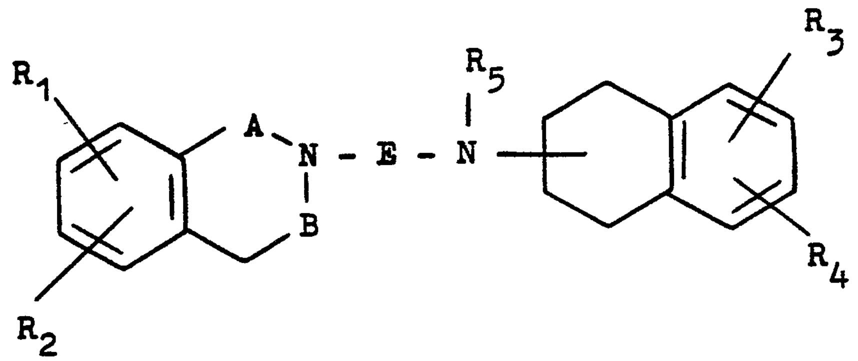

,(I)

in der

A eine $-CH_2-CH_2-$, $-CH=CH-$, $-NH-CO-$ oder $-CH_2-CO-$Gruppe und

B eine Methylen-, Carbonyl- oder Thiocarbonylgruppe oder

A eine $-CO-CO-$ oder $-\overset{OH}{CH}-CO-$Gruppe und B eine Methylengruppe,

E eine gegebenenfalls durch eine Alkylgruppe mit 1 bis 3 Kohlenstoffatomen substituierte geradkettige Alkylengruppe mit 2 bis 4 Kohlenstoffatomen, eine 2-Hydroxy-n-propylen-, 2-Hydroxy-n-butylen- oder 3-Hydroxy-n-butylengruppe,

$R_1$ ein Wasserstoff-, Fluor-, Chlor- oder Bromatom, eine Trifluormethyl-, Nitro-, Amino-, Alkylamino-, Dialkylamino-, Alkyl-, Alkylthio-, Hydroxy-, Alkoxy- oder Phenylalkoxygruppe, wobei jeder Alkylteil jeweils 1 bis 3 Kohlenstoffatome enthalten kann,

$R_2$ ein Wasserstoff-, Chlor- oder Bromatom, eine Hydroxy-, Alkoxy-, Phenylalkoxy- oder Alkylgruppe, wobei jeder Alkylteil jeweils 1 bis 3 Kohlenstoffatome enthalten kann, oder

$R_1$ und $R_2$ zusammen eine Alkylendioxygruppe mit 1 oder 2 Kohlenstoffatomen,

$R_3$ und $R_4$, die gleich oder verschieden sein können, Wasserstoff-, Fluor-, Chlor- oder Bromatome, Alkyl-, Hydroxy-, Alkoxy-, Nitro-, Amino-, Alkylamino- oder Dialkylaminogruppen oder zusammen eine Methylendioxygruppe, wobei jeder Alkylteil 1 bis 3 Kohlenstoffatome enthalten kann, und

$R_5$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatomen enthalten kann, bedeuten, und deren Säureadditionssalze mit anorganischen oder organischen Säuren.

2. Aminotetralinderivate der allgemeinen Formel

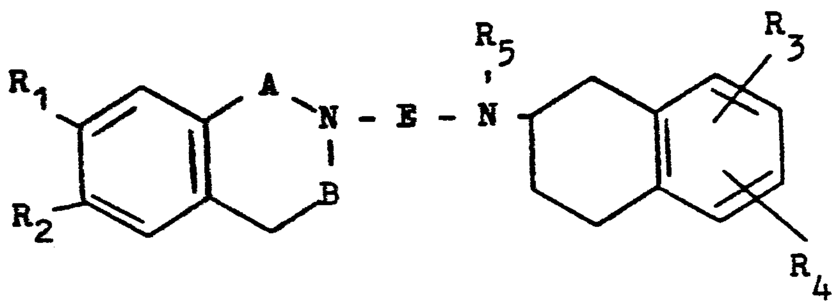

,(Ia)

in der

A eine $-CH_2-CH_2-$, $-CH=CH-$, $-NH-CO-$, $-CH_2-CO-$, $-CO-CO-$

OH

oder -CH-CO-Gruppe und B eine Methylengruppe oder

A eine $-CH_2-CH_2-$ oder -CH=CH-Gruppe und B eine Carbonyl- oder Thiocarbonylgruppe sind,

E eine n-Propylengruppe,

$R_1$ ein Chloratom- oder Bromatom, eine Methyl-, Methoxy-, Nitro-, Amino-, Methylamino- oder Dimethylaminogruppe,

$R_2$ ein Chlor- oder Bromatom, eine Methyl- oder Methoxygruppe oder $R_1$ und $R_2$ zusammen eine Methylendioxy- oder Ethylendioxygruppe,

$R_3$ ein Wasserstoff-, Chlor- oder Bromatom, eine Hydroxy-, Methoxy-, Amino-, Methylamino- oder Dimethylaminogruppe,

$R_4$ ein Wasserstoff- oder Chloratom oder eine Methoxygruppe zusammen mit $R_3$ eine Methylendioxygruppe und
und

$R_5$ ein Wasserstoffatom, eine Methyl- oder Allylgruppe bedeuten, und deren Säureadditionssalze mit anorganischen oder organischen Säuren.

3. Aminotetralinderivate der allgemeinen Formel Ia gemäß Anspruch 2, in der

A eine $-CH_2-CH_2-$ und B eine Carbonyl- oder Thiocarbonylgruppe oder

A eine -NH-CO-Gruppe und

B eine Methylengruppe,

E eine n-Propylengruppe,

$R_1$ und $R_2$ jeweils eine Methoxygruppe oder $R_1$ und $R_2$ zusammen eine Methylendioxygruppe,

0161604

$R_3$ und $R_4$ jeweils eine Methoxygruppe oder $R_3$ und $R_4$ zusammen eine Methylendioxygruppe und

$R_5$ ein Wasserstoffatom oder eine Methylgruppe bedeuten, und deren Säureadditionssalze mit anorganischen oder organischen Säuren.

4. 1-(7,8-Dimethoxy-1,3,4,5-tetrahydro-2H-3-benzazepin-2-on-3-yl)-3-[N-methyl-N-(6,7-dimethoxy-1,2,3,4-tetrahydronaphthyl-2)-amino]-propan und dessen Säureadditionssalze.

5. 1-(7,8-Dimethoxy-1,3,4,5-tetrahydro-2H-3-benzazepin-2-on-3-yl)-3-[N-(6,7-dimethoxy-1,2,3,4-tetrahydronaphthyl-2)-amino]-propan und dessen Säureadditionssalze.

6. Physiologisch verträgliche Säureadditionssalze der Verbindungen gemäß den Ansprüchen 1 bis 5 mit anorganischen oder organischen Säuren.

7. Arzneimittel, enthaltend eine Verbindung der allgemeinen Formel I gemäß den Ansprüchen 1 bis 5 oder dessen physiologisch verträgliches Säureadditionssalz gemäß Anspruch 6 neben einem oder mehreren inerten Trägerstoffen und/oder Verdünnungsmitteln.

8. Verfahren zur Herstellung eines Arzneimittels zur Behandlung von Sinustachykardien und ischämischer Herzerkrankungen, dadruch gekennzeichnet, daß auf nichtchemischem Wege eine Verbindung gemäß den Ansprüchen 1 bis 5 oder dessen physiologsich verträgliches Säureadditionssalz gemäß Anspruch 6 in einen oder mehreren inerten Trägerstoffen und/oder Verdünnungsmitteln eingearbeitet wird.

9. Verwendung einer Verbindung gemäß den Ansprüchen 1 bis 6 zur Behandlung von Sinustachykardien und ischäischen Herzerkrankungen.

 

10. Verfahren zur Herstellung von neuen Aminotetralinderivaten gemäß den Ansprüchen 1 bis 6, dadurch gekennzeichnet, daß

a) eine Verbindung der allgemeinen Formel

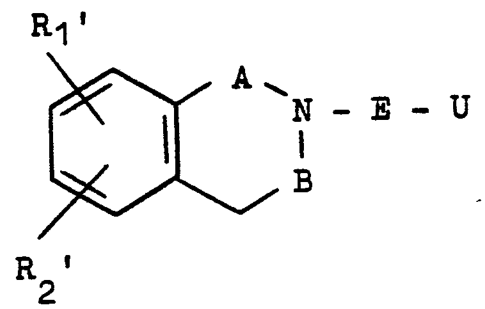

,(II)

mit einer Verbindung der allgmeinen Formel

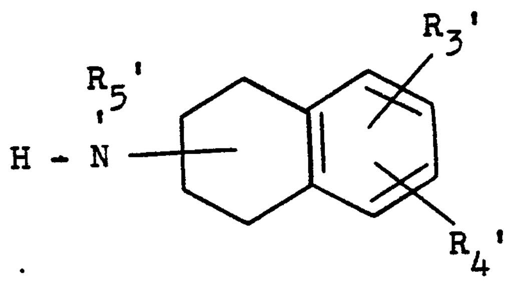

,(III)

in der
A, B und E wie eingangs definiert sind,
$R_1$' eine durch einen Schutzrest geschützte Hydroxy-, Amino- oder Alkylaminogruppe darstellt oder die für $R_1$ eingangs erwähnten Bedeutungen besitzt,
$R_2$' eine durch einen Schutzrest geschützte Hydroxygruppe darstellt oder die für $R_2$ eingangs erwähnten Bedeutungen besitzt,
$R_3$' eine durch einen Schutzrest geschützte Hydroxy-, Amino- oder Alkylaminogruppe darstellt oder die für $R_3$ eingangs erwähnten Bedeutungen besitzt,
$R_4$' eine durch einen Schutzrest geschützte Hydroxy-, Amino- oder Alkylaminogruppe darstellt oder die für $R_4$ eingangs erwähnten Bedeutungen besitzt,
$R_5$' eine Schutzgruppe für eine Aminogruppe darstellt oder die für $R_5$ eingangs erwähnten Bedeutungen besitzt, und
U eine nukleophile Austrittsgruppe wie ein Halogenatom oder eine Sulfonyloxygruppe, z.B. ein Chlor-, Brom- oder Jodatom,

die Methansulfonyloxy-, p-Toluolsulfonyloxy- oder Ethoxysulfonyloxygruppe, darstellt, umgesetzt und gegebenenfalls anschließend ein verwendeter Schutzrest abgespalten wird oder

b) zur Herstellung von Verbindungen der allgemeinen Formel I, in der A die $-CH_2-CH_2-$Gruppe, B die Methylen- oder Carbonylgruppe und $R_5$ keine Alkenylgruppe mit 3 bis 5 Kohlenstoffatomen darstellen, eine Verbindung der allgemeinen Formel

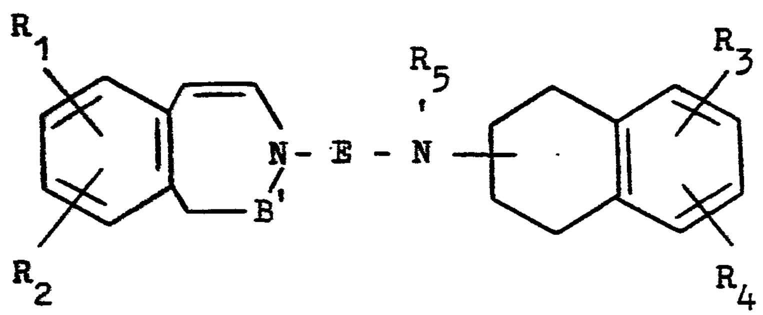

,(IV)

in der
$R_1$ bis $R_5$ und E wie eingangs definiert sind und
B' die Methylen- oder Carbonylgruppe darstellt, hydriert wird oder

c) zur Herstellung von Verbindungen der allgemeinen Formel I, in der B eine Thiocarbonylgruppe darstellt, eine Verbindung der allgemeinen Formel

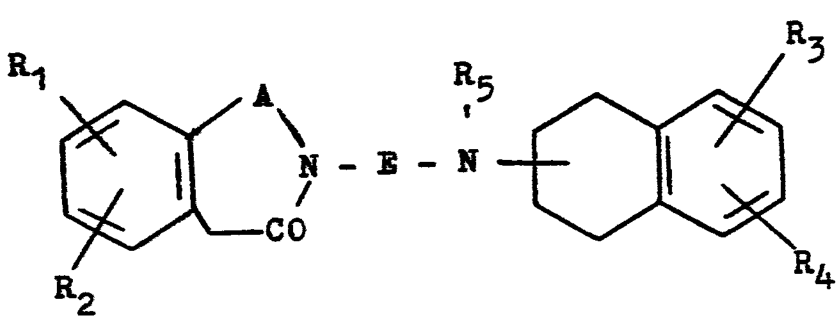

,(V)

in der
$R_1$ bis $R_5$, A und E wie eingangs definiert sind, mit einem schwefeleinführenden Mittel umgesetzt wird oder

d) zur Herstellung von Verbindungen der allgemeinen Formel I, in der A eine $-\overset{OH}{\overset{|}{C}}H-CO-$Gruppe darstellt, eine Verbindung der allgemeinen Formel

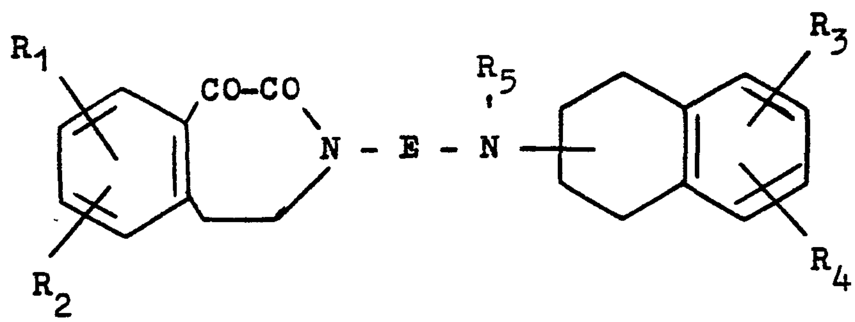

,(VI)

in der

$R_1$ bis $R_5$ und E wie eingangs definiert sind, reduziert wird oder

e) zur Herstellung von Verbindungen der allgemeinen Formel I, in der A eine $-CH_2-CH_2-$ oder $-CH=CH-$ und B eine Methylengruppe darstellen, eine Verbindung der allgemeinen Formel

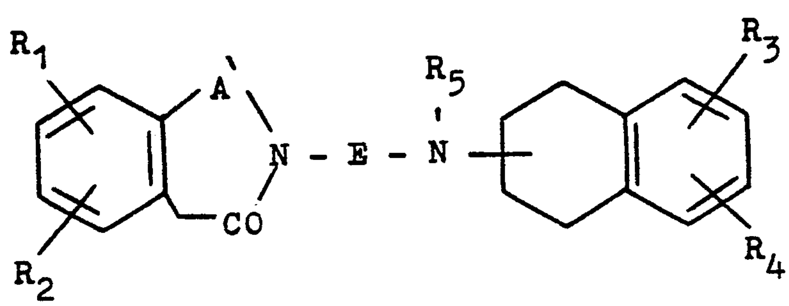

,(VII)

in der

$R_1$ bis $R_5$ und E wie eingangs definiert sind und

A' eine $-CH_2-CH_2-$ oder $-CH=CH-$Gruppe darstellt, reduziert wird oder

0161604

f) zur Herstellung von Verbindungen der allgemeinen Formel I, in der A die -COCO-Gruppe darstellt, eine Verbindung der allgemeinen Formel

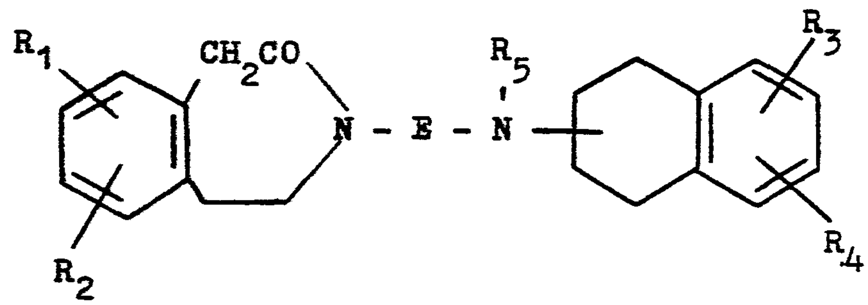

,(VIII)

in der $R_1$ bis $R_5$ und E wie eingangs definiert sind, oxidiert wird oder

g) zur Herstellung von Verbindungen der allgemeinen Formel I, in der A die -NH-CO-Gruppe und E eine gegebenenfalls durch eine Alkylgruppe mit 1 bis 3 Kohlenstoffatomen substituierte Alkylengruppe mit 2 bis 4 Kohlenstoffatomen darstellen, eine Verbindung der allgemeinen Formel

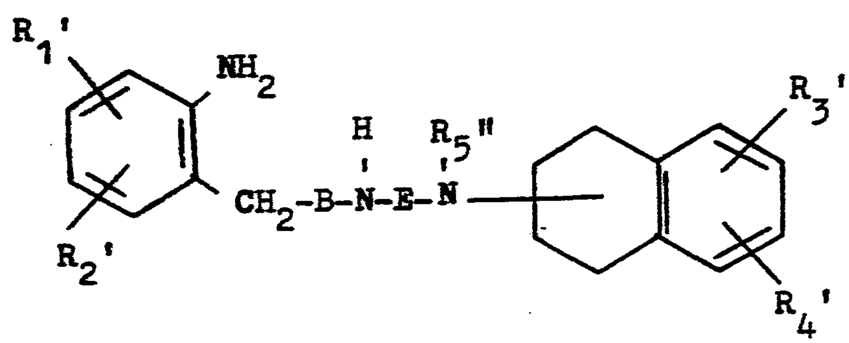

,(IX)

in der

B und E wie eingangs definiert sind,

$R_1$' eine durch eine Schutzgruppe geschützte Hydroxy-, Amino- oder Alkylaminogruppe darstellt oder die für $R_1$ eingangs erwähnten Bedeutungen besitzt,

$R_2$' eine durch eine Schutzgruppe geschützte Hydroxygruppe darstellt oder die für $R_2$ eingangs erwähnten Bedeutungen besitzt,
$R_3$' und $R_4$', die gleich oder verschieden sein können, jeweils eine durch eine Schutzgruppe geschützte Hydroxy-, Amino- oder Alkylaminogruppe darstellen oder die für $R_3$ bzw. $R_4$ eingangs erwähnten Bedeutungen besitzen,
$R_5$" eine Schutzgruppe für eine Aminogruppe darstellt oder mit Ausnahme von Wasserstoff die für $R_5$ eingangs erwähnten Bedeutungen besitzt, mit einem Kohlensäurederivat der allgemeinen Formel

$$W - CO - W \qquad ,(X)$$

in der
W, die gleich oder verschieden sein können, jeweils eine nukleophile Austrittsgruppe bedeuten, umgesetzt und gegebenenfalls anschließend ein verwendeter Schutzrest abgespalten wird oder

h) eine Verbindung der allgemeinen Formel

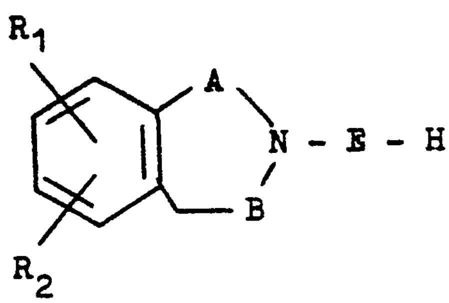

,(XI)

in der
A, B, E, $R_1$ und $R_2$ wie eingangs definiert sind, wobei jedoch im Rest E zwei Wasserstoffatome in einer $-CH_2-$ oder $CH_3$-Gruppe des Restes E durch ein Sauerstoffatom ersetzt sind, mit einer Verbindung der allgemeinen Formel

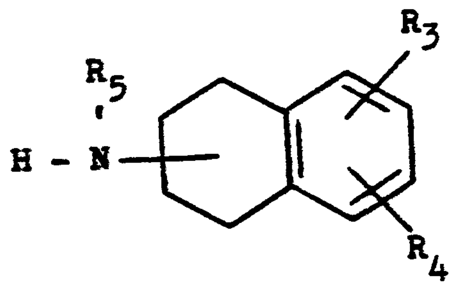

,(XII)

in der

$R_3$ bis $R_5$ wie eingangs definiert sind, in Gegenwart eines Reduktionsmittels umgesetzt wird und

gewünschtenfalls anschließend eine so erhaltende Verbindung der allgemeinen Formel I in ihr Säureadditionssalz, insbesondere in ihr physiologisch verträgliches Säureadditionssalz mit einer anorganischen oder organischen Säure übergeführt wird.